(19) Europäisches Patentamt
European Patent Office
Office européen des brevets

(11) EP 3 436 827 B1

(12) FASCICULE DE BREVET EUROPEEN

(45) Date de publication et mention de la délivrance du brevet:
08.01.2020 Bulletin 2020/02

(51) Int Cl.:
G01N 33/564 (2006.01)     G01N 33/569 (2006.01)
G01N 33/68 (2006.01)      G01N 21/552 (2014.01)
G01N 33/543 (2006.01)

(21) Numéro de dépôt: 17717789.6

(22) Date de dépôt: 28.03.2017

(86) Numéro de dépôt international:
PCT/FR2017/050702

(87) Numéro de publication internationale:
WO 2017/168083 (05.10.2017 Gazette 2017/40)

(54) **PROCÉDÉ DE DÉTERMINATION DES CONCENTRATIONS ACTIVES ET/OU DES CONSTANTES CINÉTIQUES D'INTERACTION DANS DES ÉCHANTILLONS BIOLOGIQUES COMPLEXES EN RÉSONANCE PLASMONIQUE DE SURFACE**

VERFAHREN ZUR BESTIMMUNG AKTIVER KONZENTRATIONEN UND/ODER KINETISCHER INTERAKTIONSKONSTANTEN IN KOMPLEXEN BIOLOGISCHEN PROBEN MITTELS OBERFLÄCHENPLASMONRESONANZ

METHOD FOR DETERMINING ACTIVE CONCENTRATIONS AND/OR KINETIC INTERACTION CONSTANTS IN COMPLEX BIOLOGICAL SAMPLES BY MEANS OF SURFACE PLASMON RESONANCE

(84) Etats contractants désignés:
AL AT BE BG CH CY CZ DE DK EE ES FI FR GB GR HR HU IE IS IT LI LT LU LV MC MK MT NL NO PL PT RO RS SE SI SK SM TR

(30) Priorité: 29.03.2016 FR 1652699

(43) Date de publication de la demande:
06.02.2019 Bulletin 2019/06

(73) Titulaires:
• Université de Bordeaux
  33000 Bordeaux (FR)
• Centre Hospitalier Universitaire de Bordeaux
  33404 Talence Cedex (FR)
• Centre National de la Recherche Scientifique
  75016 Paris (FR)
• INSERM (Institut National de la Santé et de la Recherche Médicale)
  75013 Paris (FR)

(72) Inventeurs:
• VISENTIN, Jonathan
  33200 Bordeaux (FR)
• DI PRIMO, Carmelo
  33700 Merignac (FR)
• TAUPIN, Jean-Luc
  75004 Paris (FR)

(74) Mandataire: Cabinet Becker et Associés
25, rue Louis le Grand
75002 Paris (FR)

(56) Documents cités:
US-A1- 2003 059 954

• VISENTIN JONATHAN ET AL: "Calibration free concentration analysis by surface plasmon resonance in a capture mode", TALANTA, vol. 148, 10 novembre 2015 (2015-11-10), pages 478-485, XP029328706, online ISSN: 0039-9140, DOI: 10.1016/J.TALANTA.2015.11.025
• Anonymous: "Guide to SPR Data Processing on the ProteOn (TM) XPR36 Protein Interaction Array System", , 2013, pages 1-8, XP055316737, Extrait de l'Internet: URL:http://www.bio-rad.com/webroot/web/pdf/lsr/literature/Bulletin_6300.pdf [extrait le 2016-11-04]

**Description**

**Domaine de l'invention**

La présente invention est relative à un procédé pour la détermination des concentrations actives, et éventuellement des constantes cinétiques d'interaction, d'un analyte dans des échantillons biologiques complexes par résonance plasmonique de surface (SPR).

**Arrière-plan technologique de l'invention**

Durant les trois premières décennies de la transplantation d'organe, les efforts ont convergé vers la mise au point de traitements immunosuppresseurs actifs sur les lymphocytes T, le principal succès étant les inhibiteurs de la calcineurine qui ont permis de réduire l'incidence des rejets aigus cellulaires et une augmentation de la survie à court terme des organes transplantés. Néanmoins, la survie des greffons à long terme n'a été que modestement améliorée. L'évolution des techniques permettant de détecter les anticorps anti-HLA a éclairé notre vision de ces pertes de greffon tardives qui semblent en majorité dues à la réponse allo-immune humorale. En effet, quel que soit l'organe, la présence d'anticorps spécifiques du donneur (DSA) anti-HLA (DSA : Donor specific antibodies) entraîne un cortège de lésions inflammatoires aiguës et/ou chroniques altérant la fonction et la survie du greffon à plus ou moins long terme. Les DSA, d'isotype IgG en particulier, sont une cause de perte de greffon majeure en transplantation d'organes.

Les DSA anti-HLA reconnaissent les molécules HLA du donneur qui sont différentes de celles du receveur en termes de séquences en acides aminés. En effet, les molécules HLA étant très polymorphiques, la plupart des transplantations d'organes sont réalisées avec des différences entre donneurs et receveurs. Ces différences sont réparties à différents endroits de la molécule HLA, de manière localisée, une grande partie de la molécule étant conservée d'un antigène à un autre.

Les tests « single antigen » Luminex® (SAFB) sont actuellement les outils les plus précis et sensibles pour l'identification des DSA IgG anti-HLA dans le sérum des receveurs. Le SAFB mesure une intensité moyenne de fluorescence (MFI), utilisée comme une valeur semi-quantitative de la « force » de l'anticorps. Au-delà du seuil de positivité biologique qui peut se définir à partir du bruit de fond moyen observé avec la technique, le seuil de positivité clinique n'est pas encore très bien défini. L'absence de matériau de référence, la disparité en termes de valeurs de MFI obtenues dans les différents laboratoires d'histocompatibilité et en fonction du fournisseur ne facilitent pas cette tâche. Ainsi, la MFI n'est pas parfaitement associée à l'issue clinique, ce qui pourrait être dû à plusieurs raisons. Premièrement, les SAFB de classe I détectent fréquemment des anticorps anti-HLA dénaturé de classe I, ceux-ci étant incapables de se lier à la surface cellulaire et n'ayant donc pas de signification clinique. Par conséquent, la détection de ces anticorps anti-HLA dénaturé de classe I a un impact négatif sur l'accès à la transplantation par surestimation erronée de l'immunisation du patient. Deuxièmement, les IgG anti-HLA de forte MFI sont capables d'activer le complément à la surface des billes, conduisant à une accumulation des produits de dégradation du C4 et du C3, capables de réduire la détection des IgG anti-HLA par encombrement stérique. Il a également été démontré que les IgM anti-HLA étaient aussi capables d'interférer avec la détection des IgG à travers une compétition pour l'épitope, un encombrement stérique et une activation du complément. Enfin, le SAFB étant une technique en point final, celui-ci ne reflète pas le mode d'interaction des DSA avec le greffon *in vivo,* et ne permet pas d'apprécier les constantes cinétiques d'interaction entre les DSA et leurs cibles, alors que ces propriétés pourraient influer sur leur pathogénicité.

La SPR est par contre une technique permettant de déterminer les constantes cinétiques d'interaction. La SPR repose sur les changements de propriétés de réflexion d'une mince couche d'or sur laquelle, d'un côté une lumière polarisée est dirigée à travers un prisme avant d'être réfléchie vers un photodétecteur, et de l'autre côté un ligand est immobilisé et baigne dans un flux de tampon. Dans des conditions de réflexion interne totale, la lumière polarisée génère une onde évanescente pénétrant dans la fine couche d'or et sur quelques centaines de nanomètres dans le tampon, où une partie des photons sera absorbée. Le photodétecteur mesure l'intensité de la lumière réfléchie, ce qui permet d'observer, à un angle donné, un minimum d'intensité correspondant à l'absorption de l'énergie de l'onde évanescente par les électrons libres de la surface. L'angle auquel se situe ce minimum est appelé angle de résonance et varie en fonction de la masse présente dans le champ de l'onde évanescente, à proximité de la couche d'or, tout changement de masse modifiant alors l'indice de réfraction. La SPR est donc une technique ne nécessitant pas de marquage préalable des molécules étudiées (« label-free »).

En pratique, la plupart des instruments de SPR utilisent des biopuces présentant une surface d'analyse séparée en plusieurs pistes ou spots. Une fois le ligand attaché à la surface, une solution contenant l'analyte à étudier est injectée à un flux constant et pendant un temps donné. L'appareil mesure en permanence et en « temps réel » la variation d'angle de résonance due à la variation de masse à la surface de la piste. Le signal mesuré en fonction du temps constitue un sensorgramme dont l'unité est l'unité de résonance (« resonance unit » ou RU). Une RU correspond à la fixation d'un ng de protéine par mm$^2$. En l'absence d'injection de solution à tester, un tampon de course circule sur la surface et le signal ne varie pas en fonction du temps. Durant l'injection d'un analyte capable de se fixer au ligand immobilisé, la masse augmente à la surface de la piste. L'angle de résonance varie car l'indice de réfraction change. Le signal mesuré (RU) augmente en fonction du temps. Ainsi, il est possible d'étudier en temps réel l'interaction d'un analyte avec un ligand donné. A noter, la variation de l'angle de résonance lors de la formation du complexe dépend du rapport des masses entre ces deux derniers. De plus, avant de pouvoir interagir avec le ligand qui est immobilisé dans la matrice de polymère, l'analyte doit diffuser depuis le flux (ou bulk) passant au-dessus de cette matrice vers cette matrice selon une constante $k_m$ qui est le coefficient de transport de masse.

Le $k_m$ dépend du coefficient de diffusion de l'analyte (D), du flux d'injection (f) et des dimensions de la cellule de mesure. Le coefficient de diffusion de l'analyte dépend de sa masse moléculaire, de son coefficient de friction ainsi que de la viscosité relative de la solution utilisée.

A la fin de l'injection, le tampon de course prend le relais, l'analyte se dissocie et est entraîné dans le flux de tampon. La masse diminuant à la surface de la piste, l'angle de résonance varie dans l'autre sens. Le signal (RU) diminue alors en fonction du temps. Cette phase de dissociation est plus ou moins rapide, selon la vitesse de dissociation du complexe, indépendamment de la vitesse du flux de tampon.

Pour la plupart des couples antigène-anticorps (Ag-Ac), la vitesse de dissociation du complexe est très lente. Ceci nécessite d'injecter une solution de régénération sur la surface afin de dissocier instantanément le complexe pour pouvoir réaliser un nouveau cycle d'injection de l'analyte. La solution de régénération est souvent constituée d'une solution acide ou basique, d'une solution de force ionique importante ou contenant des détergents et des composés organiques. Cette solution doit dissocier de manière totale et instantanée les complexes analyte-ligand sans dégrader le ligand immobilisé ni la surface de la biopuce. Ceci permet de multiplier les injections sur la surface sans avoir à changer de piste ni à ré-immobiliser une quantité identique de ligand.

Dans le cadre de l'analyse des anticorps HLA, l'immobilisation directe (covalente) de la molécule HLA sur la biopuce n'est pas envisageable car la molécule HLA ne résiste pas à la régénération. Un système de capture a donc été mis au point et validé pour permettre de contourner cette difficulté (Visentin et al., 2016, Talanta, 148, 478-485). Ainsi, un anticorps anti-β2-microglobuline (chaîne invariable associée à toutes les molécules HLA de classe I) est immobilisé de manière covalente sur la surface et sert à capturer les molécules HLA, représentant le ligand, qui peuvent ensuite être le siège de l'interaction avec les anticorps anti-HLA, l'analyte.

La SPR permet de déterminer la concentration active d'un analyte, c'est-à-dire la concentration de la fraction d'analyte capable d'interagir avec le ligand. Contrairement à d'autres méthodes, cette mesure peut être réalisée sans courbe de calibration. La méthode, connue sous le nom de *calibration-free concentration analysis* ou CFCA, repose sur le phénomène de limitation de transport de masse décrit précédemment. Si la vitesse de diffusion de l'analyte vers la surface est plus lente que la vitesse d'association à son ligand, la vitesse d'association apparente se trouvera diminuée car elle sera dépendante de la diffusion de l'analyte vers sa cible. Pour un analyte utilisé dans les mêmes conditions de concentration, température et solution, le coefficient de transport de masse ($k_m$) ne dépendra alors que de la vitesse du flux. On observera alors une pente d'association initiale différente en fonction du flux.

Il a ainsi été montré que la SPR utilisée en mode capture (Visentin et al., 2016, Talanta, 148, 478-485) permettait de déterminer la concentration active et les constantes cinétiques d'interaction d'anticorps anti-HLA (Human Leucocyte Antigens) monoclonaux, dans un milieu simple (tampon physiologique). A ce jour, c'est la seule méthode capable de le faire.

L'utilisation de la CFCA est primordiale dans le contexte des anticorps anti-HLA en provenance de patients étant donné qu'il est par définition impossible de réaliser une courbe de calibration. Chaque individu ayant a priori des anticorps uniques en termes d'affinité avec leur cible HLA, il n'existe pas de matériau de référence.

L'utilisation d'un flux lent, d'une grande quantité de ligand immobilisé et d'une concentration en analyte faible favorisent la limitation par le transport de masse. Dans le cadre de cette application en mode capture, cela nécessite d'immobiliser une très grande quantité d'anticorps de capture sur la surface. L'analyte est alors injecté selon deux flux différents, un lent et un rapide. La comparaison des vitesses d'association en fonction du flux utilisé permet de calculer la concentration active de l'analyte.

La SPR permet également de déterminer l'affinité du complexe analyte-ligand en injectant des concentrations croissantes d'analyte sur la surface. Les expériences peuvent être réalisées en plusieurs cycles d'association - dissociation puis régénération [analyse cinétique en cycles multiples ou *multiple cycle kinetics analysis* (MCK)], ou en injectant des concentrations croissantes de l'analyte jusqu'à saturation de la cible, sans cycle de régénération entre les injections [analyse cinétique à cycle unique ou *single cycle kinetics analysis* (SCK)]. Ces deux méthodes ont déjà été utilisées en mode capture.

Les logiciels d'analyse permettent de réaliser un ajustement ou « fit » des sensorgrammes suivant un modèle d'interaction donné, le plus souvent un modèle bimoléculaire simple 1:1. Les constantes élémentaires d'association et de dissociation, $k_a$ et $k_d$, respectivement, sont déterminées directement à partir de cette analyse.

Il est important de noter que la détermination de ces paramètres nécessite de connaître précisément la concentration active de l'analyte, c'est-à-dire la concentration de la fraction de l'échantillon réellement capable d'interagir avec le ligand. En effet, la connaissance de la concentration de l'analyte injecté est nécessaire pour analyser les données cinétiques. Dans le cadre de l'étude de couples Ag-Ac, la détermination de la concentration de l'anticorps par des techniques spectrophotométriques ou colorimétriques est grossière car ces techniques ne peuvent mesurer qu'une concentration totale. Elles ne s'affranchissent pas des impuretés qui résultent de la purification et qui contribueront à la valeur mesurée. Elles ne permettent pas non plus d'estimer la fraction d'anticorps dénaturés lors de la purification.

Malheureusement, la méthode décrite dans Visentin et al., 2016 (Talanta, 148, 478-485) n'est pas directement utilisable sur des milieux complexes comme par exemple les sérums/plasmas de patients et leurs dérivés. En effet, l'application de cette méthode se heurte à des problèmes techniques liés à la fixation non spécifique (« NSB » pour non-specific binding) de divers constituants plus ou moins bien connus ou inconnus de ce type d'échantillons sur la surface d'analyse. Ces problèmes de fixation non-spécifique rendent impossible l'interprétation des résultats.

Ainsi, malgré le besoin existant de déterminer et caractériser de manière fine et précise les anticorps anti-HLA dans des échantillons biologiques complexes, la méthode SPR n'est pas applicable à ce type d'échantillon. Ce besoin peut être élargi à d'autres couples analyte-ligand pour lesquels les mêmes difficultés sont rencontrées lorsque l'on considère des échantillons complexes.

## Résumé de l'invention

L'objet de l'invention permet de résoudre les problèmes techniques rencontrés lors de l'application du procédé de détermination de la concentration active d'un analyte et éventuellement les constantes cinétiques d'interaction de l'analyte avec un ligand à des milieux complexes tels que du sérum ou du plasma humains. Deux ans de développements ont été nécessaires à l'obtention d'un procédé adapté.

L'objet de l'invention est donc un mode particulier de mise en œuvre de la technique SPR en mode capture permettant d'obtenir les paramètres de concentration active et de constantes cinétiques d'interaction pour des échantillons complexes, et ainsi de répondre aux besoins cliniques et de recherche existants.

La présente invention est relative à une méthode pour déterminer dans des échantillons biologiques complexes en résonance plasmonique de surface la concentration active d'un analyte et, facultativement, des constantes cinétiques d'interaction de l'analyte avec un ligand comprenant

- la fourniture d'une puce de résonance plasmonique de surface sur laquelle est immobilisé un agent de capture spécifique d'un ligand de l'analyte ;
- la capture par l'agent de capture d'un ligand témoin ne liant pas l'analyte à tester ;
- le passage de l'échantillon sur la puce injecté à un flux déterminé pendant un temps déterminé ;
- la régénération de la surface;
- la capture par l'agent de capture d'un ligand liant l'analyte à tester ;
- le passage de l'échantillon sur la puce injecté à un même flux déterminé pendant un même temps déterminé ;
- la soustraction du sensorgramme obtenu avec le ligand témoin du sensorgramme obtenu avec le ligand liant l'analyte à tester ; et
- le calcul de la concentration active de l'analyte
- facultativement, la détermination des constantes cinétiques d'interaction de l'analyte avec le ligand,

et caractérisée en ce que le ligand témoin et le ligand liant l'analyte à tester sont de masse similaire et le ligand témoin et le ligand liant l'analyte à tester sont capturés en quantité équivalente par l'agent de capture.

De préférence, l'échantillon biologique complexe est sélectionné parmi le sérum, le plasma, les urines, les liquides de lavages, une ascite, les éluats de biopsies et les milieux de culture cellulaire. Dans un mode de réalisation préféré, l'échantillon biologique complexe est le sérum ou le plasma.

Dans un premier mode de réalisation, l'échantillon est injecté à au moins deux flux différents et la concentration active de l'analyte dans l'échantillon biologique complexe est calculée. En outre, l'échantillon peut être injecté à différentes concentrations et les constantes cinétiques d'interaction de l'analyte avec le ligand dans l'échantillon biologique complexe sont calculées.

De préférence, le couple analyte-ligand est choisi parmi un couple anticorps-antigène, un couple ligand/récepteur, un couple xénobiotique/cible moléculaire.

Dans un mode de réalisation particulier, l'analyte est un anticorps anti-HLA, le ligand témoin est un antigène HLA non reconnu par l'anticorps à tester et le ligand liant l'analyte à tester est un antigène HLA cible de l'anticorps à tester. L'échantillon peut subir un ou des traitements préalables sélectionnés parmi un traitement thermique, un traitement au dithiothréitol (DTT), une étape de purification des IgG sur une résine de protéine G, une étape de concentration de l'échantillon, une étape de dialyse, en particulier avec un seuil de coupure (cut-off) de 100 kDa, et une combinaison de plusieurs de ces traitements. De préférence, l'échantillon subit préalablement une combinaison d'un traitement thermique et d'une étape de purification des IgG sur une résine de protéine G. De préférence, la méthode comprend une étape préalable dans laquelle les anticorps anti-HLA dans l'échantillon sont détectés.

Décrit ici est aussi un kit comprenant une puce de résonance plasmonique de surface comprenant au moins deux ou trois pistes choisies parmi les pistes suivantes :

- une piste sur laquelle est immobilisé un agent de capture spécifique des antigènes HLA de classe I, par exemple spécifique de la β2 microglobuline,
- une piste sur laquelle est immobilisé un agent de capture spécifique des antigènes HLA-DQ (classe II) ;
- une piste sur laquelle est immobilisé un agent de capture spécifique des antigènes HLA-DR (classe II) ; et
- une piste sur laquelle est immobilisé un agent de capture spécifique des antigènes HLA-DP (classe II).

### Description détaillée de l'invention

Comme la méthode décrite dans Visentin et al., 2016 (Talanta, 148, 478-485) n'est pas utilisable sur des milieux complexes comme par exemple les sérums/plasmas de patients et leurs dérivés, les inventeurs se sont attachés à la mise au point de cette méthode pour des échantillons complexes. En effet, l'application de cette méthode se heurte à des problèmes techniques liés à la fixation non spécifique (« NSB » pour non-specific binding) de divers constituants plus ou moins bien connus ou inconnus de ce type d'échantillons sur la surface d'analyse. Par fixation non spécifique est entendue la fixation non-désirée de molécules autres que l'analyte sur le ligand. Ces problèmes de fixation non-spécifique rendent impossible l'interprétation des résultats.

L'objet de l'invention a été de découvrir les solutions techniques capables de corriger le NSB. Il devient alors possible de déterminer la concentration active de l'analyte puis les constantes cinétiques d'interaction entre l'analyte et le ligand et par déduction la constante d'affinité pour leur cible des analytes présents dans des milieux complexes tels que des liquides biologiques dérivant de sujets humains. L'invention est également utilisable avec d'autres milieux pouvant générer du NSB tels que des surnageants de culture cellulaire contenant les anticorps à étudier. Il faut noter que la détermination des constantes cinétiques d'interaction nécessite de connaître la concentration active et que la concentration active est en soi un paramètre pouvant être utile de manière indépendante des constantes cinétiques. L'objet de l'invention est l'aboutissement de deux années d'expériences destinées à pouvoir analyser des milieux complexes tels que des échantillons de patients, donc à éliminer le NSB qu'ils génèrent systématiquement.

Les inventeurs ont découvert que chaque échantillon doit être utilisé comme son propre blanc sur une piste sur laquelle une molécule HLA non reconnue par les anticorps présents dans l'échantillon est capturée en quantité équivalente à la molécule HLA cible de ces anticorps. Ceci permet une correction systématique du NSB et l'obtention de résultats fiables. Ce test sera désigné comme l'auto-blanc. Cette approche a été testée par les inventeurs et montrée comme efficace sur des sérums de patients.

Cette méthode développée pour l'analyse des anticorps anti-HLA dans des échantillons complexes est applicable à l'analyse d'autres couples analyte-ligand dans des échantillons complexes. Notamment, cette méthode est applicable à l'analyse de couples analyte-ligand choisis parmi un couple anticorps-antigène, un couple ligand/récepteur, un couple xénobiotique/cible moléculaire.

La présente invention est donc relative à une méthode permettant de déterminer la concentration active d'un analyte et/ou les constantes cinétiques d'interaction entre un analyte et un ligand dans un échantillon complexe, la méthode comprenant :

- la fourniture d'une puce de résonance plasmonique de surface sur laquelle est immobilisé un agent de capture spécifique d'un ligand de l'analyte ;
- la capture par l'agent de capture d'un ligand témoin ne liant pas l'analyte à tester ;
- le passage de l'échantillon sur la puce injecté à un flux déterminé pendant un temps déterminé ;
- la régénération de la surface;
- la capture par l'agent de capture d'un ligand liant l'analyte à tester ;
- le passage de l'échantillon sur la puce injecté à un même flux déterminé pendant un même temps déterminé ;
- la soustraction du sensorgramme obtenu avec le ligand témoin du sensorgramme obtenu avec le ligand liant l'analyte à tester ; et
- le calcul de la concentration active de l'analyte,
- facultativement, la détermination des constantes cinétiques d'interaction de l'analyte avec le ligand, en utilisant la même surface après modification des paramètres de capture des ligands et d'injection de l'analyte,

et caractérisée en ce que le ligand témoin et le ligand liant l'analyte à tester sont de masse similaire et le ligand témoin et le ligand liant l'analyte à tester sont capturés en quantité équivalente par l'agent de capture.

Par « agent de capture spécifique d'un ligand de l'analyte » est désignée une molécule capable d'établir une liaison spécifique avec un ligand qui est lui-même capable d'interagir spécifiquement avec l'analyte.

Par « ligand témoin ne liant pas l'analyte à tester » est désignée une molécule capable d'établir une liaison spécifique avec l'agent de capture mais incapable d'établir une liaison spécifique avec l'analyte à tester.

Par « ligand liant l'analyte à tester » est désignée une molécule capable d'établir une liaison spécifique avec l'agent de capture et avec l'analyte à tester. Ce ligand peut être également désigné dans la présente demande en tant que ligand cible.

Des exemples pratiques illustrant ces désignations sont les suivants.

Dans le contexte d'un couple analyte-ligand qui est un couple anticorps-antigène, l'agent de capture est une molécule ayant une capacité de liaison à l'antigène, par exemple un anticorps spécifique de l'antigène, le ligand témoin est une molécule capable d'être liée par l'agent de capture mais pas par l'anticorps, le ligand est l'antigène, et l'analyte est l'anticorps spécifique de l'antigène. Par exemple, l'agent de capture peut être également un aptamère oligonucléotidique. Les aptamères sont en général des composés synthétiques, isolés in vitro à partir de banques combinatoires d'un grand nombre de composés de séquence aléatoire par une méthode de sélection itérative appelée SELEX.

Dans le contexte d'un couple analyte-ligand qui est un couple récepteur-ligand, l'agent de capture est une molécule ayant une capacité de liaison au ligand, par exemple un anticorps spécifique du ligand, le ligand témoin est une molécule capable d'être liée par l'agent de capture mais pas par le récepteur, le ligand est le ligand du récepteur, et l'analyte est le récepteur spécifique du ligand, ou inversement, le ligand peut être le récepteur et l'analyte un ligand du récepteur.

Dans le contexte d'un couple analyte-ligand qui est un couple xénobiotique-cible moléculaire, l'agent de capture est une molécule ayant une capacité de liaison à la cible moléculaire, par exemple un anticorps spécifique de cette cible, le ligand témoin est une molécule capable d'être liée par l'agent de capture mais pas par le xénobiotique, le ligand est la cible moléculaire, et l'analyte est le xénobiotique.

Par spécifique est entendue une fixation préférentielle par rapport à d'autres, de préférence par un facteur d'au moins 100, 1000 ou 10 000.

Par « sensorgramme » est entendue la représentation (graphique) du signal SPR mesuré en fonction du temps, correspondant à la variation de l'angle de résonance due à la variation de masse dans le champ de l'onde évanescente. Le signal de résonance est exprimé en unités de résonance (RU). De préférence, les sensorgrammes sont mesurés avec une machine Biacore adaptée ou équivalent, notamment le Biacore T200.

Lorsqu'il est précisé que le ligand témoin et le ligand liant l'analyte à tester sont de masse similaire, cela signifie que les ligands, lorsqu'ils se fixent à l'agent de capture, engendrent un signal substantiellement identique. Ainsi, dans un mode de réalisation particulier, leurs masses ne diffèrent de pas plus de 10%, de préférence de pas plus de 5%. Ainsi, pour un ligand liant l'analyte à tester de 100 kDa, la masse du ligand témoin sera comprise entre 90-110 kDa, de préférence entre 95-105 kDa.

Lorsqu'il est précisé « capturées en quantité équivalente par l'agent de capture », cela signifie que la quantité de molécule capturée, de préférence mesurée en RU, est la même à plus ou moins 20%, de préférence à plus ou moins 15, de manière encore plus préférée à plus ou moins 10%. La quantité de molécule capturée est de préférence mesurée en RU.

Il est à noterque l'étape d'auto-blanc en présence du ligand témoin peut être réalisée avant ou après l'étape de test de l'analyte avec le ligand cible.

Dans le contexte spécifique où l'analyte est un anticorps anti-HLA, la présente invention est donc relative à une méthode permettant de déterminer la concentration active et/ou les constantes cinétiques d'interaction d'anticorps anti-HLA à tester avec l'antigène HLA reconnu par l'anticorps dans un échantillon complexe, la méthode comprenant :

- la fourniture d'une puce de résonance plasmonique de surface sur laquelle est immobilisé un agent de capture spécifique des antigènes HLA reconnus par l'anticorps ;
- la capture ou immobilisation par l'agent de capture d'un antigène HLA non reconnu par l'anticorps à tester ;
- le passage de l'échantillon sur la puce injecté à un flux déterminé pendant un temps déterminé ;
- la régénération de la surface ;
- la capture par l'agent de capture d'un antigène HLA cible de l'anticorps à tester ;
- le passage de l'échantillon sur la puce injecté à un même flux déterminé pendant un même temps déterminé ;
- la soustraction du sensorgramme obtenu avec l'antigène HLA non reconnu par l'anticorps anti-HLA du sensorgramme obtenu avec l'antigène HLA reconnu par l'anticorps anti-HLA ; et
- le calcul de la concentration active de l'anticorps reconnaissant l'antigène HLA
- facultativement, la détermination des constantes cinétiques d'interaction de l'analyte avec le ligand, en utilisant la même surface après modification des paramètres de capture des ligands et d'injection de l'analyte,

et caractérisée en ce que l'antigène HLA non reconnu par l'anticorps à tester et l'antigène HLA cible de l'anticorps à tester sont capturés en quantité équivalente par l'agent de capture.

Il est à noter que l'étape d'auto-blanc peut être réalisée avant ou après l'étape de test de l'anticorps anti-HLA avec l'antigène HLA cible.

Par « HLA cible de l'anticorps » est entendu l'antigène HLA reconnu par l'anticorps anti-HLA.

La méthode concerne tout particulièrement des anticorps anti-HLA. De préférence, les anticorps sont des IgG, notamment des IgG1, IgG2, IgG3 et/ou IgG4. Plus particulièrement, la méthode s'applique aux IgG par rapport aux IgM.

Puce

La puce est un support solide adapté à la SPR. De telles puces sont bien connues de l'homme du métier. La surface active est constituée d'une matrice de polymères (dextran) fixée sur une couche d'or par l'intermédiaire d'un linker (lien chimique). De préférence, la puce comprend une couche de dextran carboxyméthylée attachée de manière covalente à la fine couche d'or déposée sur la surface. La matrice permet d'immobiliser de manière covalente l'agent de capture grâce à une chimie de couplage simple utilisant par exemple les groupements amines, thiols, hydroxyles, carboxyles ou aldéhydes. Il est également possible d'utiliser un système non covalent, c'est-à-dire de capture, par exemple en immobilisant un anticorps reconnaissant l'agent de capture (par exemple un anticorps anti IgG de souris si l'agent de capture est une IgG de souris), ou bien en immobilisant de la streptavidine ou des conjugués de nickel pour lier l'agent de capture qui aura préalablement été fonctionnalisé avec de la biotine ou une « étiquette » histidine, respectivement.

Diverses puces compatibles sont disponibles commercialement (GE Healthcare, Xantec Bioanalytics). De préférence, la puce est une puce CM5 vendue par GE Healthcare.

Agent de capture

La puce de SPR présente un agent de capture immobilisé sur la surface. Cet agent de capture est capable de fixer un ligand spécifique de l'analyte. L'agent de capture peut se lier directement au ligand, par exemple être un anticorps spécifique du ligand. De manière alternative, l'agent de capture peut se lier indirectement au ligand, par exemple par l'intermédiaire d'une étiquette (tag) attachée au ligand et via un anticorps dirigé contre cette étiquette. Les étiquettes peuvent par exemple être des étiquettes poly-histidine, glutathion S-transferase, ou équivalent.

L'agent de capture peut être un anticorps ou un dérivé d'un anticorps comme un anticorps Fv, un anticorps à domaine unique (dsFv ou nanobody), un anticorps simple chaîne (scFv), un Fab, un F(ab')2, un Fab', un sc(Fv)2, ou un aptamère oligonucléotidique. Cet agent de capture est immobilisé sur la puce par une chimie de couplage ou une liaison non-covalente du type biotine-streptavidine.

Dans le contexte particulier des anticorps anti-HLA, l'agent de capture est spécifique de l'antigène HLA pour lequel les anticorps à tester dans l'échantillon ont une affinité.

Lorsque l'anticorps à tester est un anticorps dirigé contre une molécule HLA de classe I, l'agent de capture est spécifique de cette classe HLA. Il peut par exemple être spécifique de la $\beta$2 microglobuline ($\beta$2M) ou d'un locus HLA de classe I. Des anticorps commerciaux spécifiques de la molécule HLA de classe I, et de préférence de la $\beta$2 microglobuline humaine, sont disponibles, par exemple le clone B2M-01 (ThermoFisher Scientific, Rockford, IL), le clone B2M-02 (Abcam, France), le clone # 883028 (R&D Systems, France) ou le clone W6/32.

Lorsque l'anticorps à tester est un anticorps dirigé contre une molécule HLA de classe II, l'agent de capture est spécifique de cette classe ou d'un locus de HLA de classe II. Il peut par exemple être spécifiquement dirigé contre les antigènes HLA-DR, HLA-DQ et/ou HLA-DP. Des anticorps commerciaux spécifiques sont disponibles. Notamment, il existe des anticorps présentant des spécificités croisées permettant de reconnaître la plupart des HLA de classe II comme l'anticorps du Clone REA332 (Miltenyi Biotec) ou le clone Tu39 (BD Biosciences, France). De manière alternative, la méthode peut mettre en œuvre des anticorps pan-DQ comme le clone Tu169 (BD Biosciences, France) ou le clone H1456 (One Lambda, Inc, Canoga Park, CA). De la même manière, pour les antigènes HLA-DR, un anticorps anti-HLA-DR sera mise en œuvre. Des exemples de tels anticorps incluent le clone G46-6 (BD Bioscience), le clone H1458 (One Lambda) ou le clone L243 (Biolegend). Enfin, pour les antigènes HLA-DP, un anticorps anti-HLA-DP sera mise en œuvre. Des exemples de tels anticorps incluent le clone B7/21 (Abcam), le clone HI43 (AbD Serotec), ou le clone SP228 (Lifespan Bioscience).

Les techniques d'immobilisation des agents de capture sur la surface de la puce sont bien connues. L'immobilisation est de préférence via une liaison covalente. Par exemple, le couplage peut être réalisé via une liaison amide en présence d'un mélange de N-hydroxysuccinimide et de N-éthyl-N'-diméthylaminopropyl carbodiimide, en suivant notamment les instructions du fournisseur (GE Healthcare). Ce mode de réalisation dans lequel l'agent de capture est immobilisé de manière covalente présente l'avantage d'obtenir une surface régénérable. De manière alternative, l'agent de capture peut être immobilisé via le couple streptavidine-biotine.

Dans un mode de réalisation, l'agent de capture est immobilisé sur la surface en une quantité suffisante. Notamment, lorsque l'agent de capture est un anticorps, il peut être immobilisé de manière à atteindre au minimum 10000 RU, de préférence au minimum 15 000 RU, par exemple entre 15 000 et 25 000 RU.

Echantillons

Les échantillons considérés par la présente invention sont des échantillons biologiques complexes. Notamment, sont considérés comme échantillons biologiques complexes des échantillons sanguins comme le sérum et le plasma, les urines, les liquides de lavages comme les lavages Broncho-Alvéolaires, une ascite, ou encore les éluats de biopsies et les milieux de culture cellulaire. Dans un mode de réalisation préféré, l'échantillon complexe est un sérum, en particulier un sérum humain.

Avant l'étude de l'échantillon par SPR, l'échantillon peut subir des traitements préalables. Notamment, l'échantillon peut subir un traitement thermique, un traitement au dithiothréitol (DTT), une étape de purification des IgG sur une résine de protéine G, une étape de concentration de l'échantillon, une étape de dialyse, en particulier avec un seuil de coupure (cut-off) adapté à l'analyte à tester, et une combinaison de plusieurs de ces traitements.

Dans un mode de réalisation préféré lorsque l'analyte est un anticorps, en particulier un anticorps anti-HLA d'isotype IgG, l'échantillon est traité par une combinaison d'un traitement thermique et une étape de purification des IgG. Dans un mode de réalisation préféré alternatif, l'échantillon est traité par une combinaison d'un traitement thermique, un traitement au dithiothréitol (DTT), et une étape de dialyse avec un seuil de coupure à 100 kDa.

Par exemple, le traitement thermique peut comprendre un traitement à la chaleur consistant en une incubation de l'échantillon à 40-70°C, de préférence 50-60°C, en particulier 56°C, pendant 10 - 60 minutes, de préférence 20-40 minutes, en particulier 30 minutes.

Le traitement au DTT peut consister en l'ajout de DTT à l'échantillon afin d'atteindre une concentration en DTT de 1-10 mM, de préférence 5 mM, puis un chauffage à 30-40°C, de préférence 37°C pendant 10 - 60 minutes, de préférence 20-40 minutes, en particulier 30 minutes.

La purification des IgG peut être notamment réalisée sur des billes de Sépharose couplées à de la protéine G (ThermoFisher Scientific) ou tout autre dispositif équivalent selon les recommandations du fournisseur. La concentration des purifiats peut être réalisée à l'aide de dispositifs de centrifugation Amicon Ultra-4 10 kDa (ThermoFisher Scientific) ou tout autre dispositif équivalent, par exemple à deux reprises afin de revenir à un volume proche du volume initial.

La dialyse des échantillons peut être réalisée contre le tampon de course à l'aide de dispositifs de dialyse Float-A-Lyzer G2 de cut-off 100 kDa (Spectrum Laboratories, Rancho Dominguez, CA) ou tout autre dispositif équivalent.

Les échantillons peuvent également être dilués ou concentrés si nécessaire. Notamment, ils peuvent être utilisés dans la méthode avec plusieurs taux de dilution différents.

De préférence, les échantillons sont préparés dans le tampon de course. De préférence, le tampon de course est un tampon phosphate pouvant comprendre un détergent. En particulier, le tampon phosphate peut être à 0,01 M et contenir du TWEEN® 20 (0,05%). Dans un mode de réalisation particulier, le tampon de course est le PBS-T (Sigma-Aldrich) à 0,05%.

Capture par l'agent de capture du ligand

Pour réaliser l'auto-blanc, la surface doit être chargée, via l'agent de capture, avec un ligand qui n'est pas lié par l'analyte à tester. Pour réaliser la mesure de l'analyte à tester, la surface doit être chargée, via l'agent de capture, avec le ligand cible de l'analyte. Dans un premier mode de réalisation lorsque l'analyte est un anticorps anti-HLA, l'anticorps anti-HLA reconnaît un HLA de classe I. Les HLA de classe I comprennent une chaîne lourde, une β2 microglobuline (β2M) et un peptide. Les HLA de classe I comprennent trois grandes catégories : les HLA-A, HLA-B et HLA-C.

Dans un deuxième mode de réalisation lorsque l'analyte est un anticorps anti-HLA, l'anticorps anti-HLA reconnaît un HLA de classe II. Les HLA de classe II comprennent deux chaînes α et β et un peptide. Les HLA de classe II comprennent trois grandes catégories : les HLA-DR, HLA-DQ et HLA-DP.

Ainsi, pour réaliser l'auto-blanc, la surface doit être chargée, via l'agent de capture, avec un antigène HLA qui n'est pas reconnu par l'anticorps à tester. Pour réaliser la mesure de l'anticorps à tester, la surface doit être chargée, via l'agent de capture, avec l'antigène HLA cible, c'est-à-dire celui reconnu par l'anticorps à tester.

Ainsi, la méthode comprend de préférence une étape préalable dans laquelle les anticorps anti-HLA sont détectés dans l'échantillon. Par exemple, la technique de Single Antigen en format Luminex® (SAFB) pourra être mise en œuvre ou toute autre méthode équivalente, par exemple un ELISA. Ainsi, à l'issue de cette étape préliminaire, la nature des anticorps anti-HLA contenus dans l'échantillon est connue et on peut déterminer quels sont les antigènes HLA reconnus par les anticorps anti-HLA présents dans l'échantillon. Par ailleurs, la méthode peut également comprendre une étape préliminaire de dosage des IgG totales. Pour cela, on peut mettre par exemple en œuvre un dosage en immuno-néphélémétrie sur un automate BNII (Siemens Healthcare Diagnostics, Marburg, Germany). De préférence, le dosage des IgG totales peut être réalisé avant et après traitement de l'échantillon.

De préférence, l'antigène HLA utilisé pour réaliser l'auto-blanc est de même classe que l'antigène reconnu par l'anticorps. En effet, il faut qu'il soit reconnu par l'agent de capture.

Ainsi, si l'anticorps anti-HLA à tester est un anticorps reconnaissant un antigène HLA de classe I, l'antigène capturé par l'agent de capture pour réaliser l'auto-blanc sera également un antigène de classe I, qui sera par exemple reconnu par un agent de capture spécifique de la β2 microglobuline. Par exemple, si l'anticorps anti-HLA à tester est un anticorps spécifique de HLA-A11, l'antigène utilisé pour l'auto-blanc peut être HLA-A2.

De manière alternative, si l'anticorps anti-HLA à tester est un anticorps reconnaissant un antigène HLA de classe II, l'antigène capturé par l'agent de capture pour réaliser l'auto-blanc sera également un antigène de classe II. Par exemple, si l'anticorps anti-HLA à tester est un anticorps spécifique de HLA-DQ2, l'antigène utilisé pour l'auto-blanc peut être HLA-DQ7 ou DQ5.

Détermination de la concentration active de l'analyte

L'analyse par la méthode de CFCA est bien connue de l'homme du métier et a notamment été décrite dans les articles Karlsson et al (1993, J. Immunol. Methods, 166, 75-778) et Sigmundsson et al (2002, Biochemistry, 41, 8263-8276). La méthode pour déterminer la concentration active par la méthode de CFCA a notamment été décrite dans la demande WO2013/002717 (incorporé ici par référence). Cette méthode est mise en œuvre dans le système commercial Biacore® (commercialisé par GE Healthcare, Uppsala, Suède).

En bref, dans les mêmes conditions de concentrations, de température et de solution, le signal SPR est mesuré en fonction du temps à deux flux différents d'injection de l'échantillon à doser. L'analyse de la vitesse initiale d'association, différente pour chaque flux, permet de déterminer la concentration active de l'échantillon injecté.

Pour la réalisation du sensorgramme, l'échantillon est passé sur la puce. Plus particulièrement, il est injecté avec un flux déterminé pendant un temps déterminé. Notamment, il faut que la vitesse de diffusion de l'analyte vers la surface soit plus lente que la vitesse d'association de l'analyte au ligand cible. Dans le contexte d'un analyte étant un anticorps anti-HLA, il faut que la vitesse de diffusion de l'anticorps vers la surface soit plus lente que la vitesse d'association à l'antigène HLA cible. Dans ce contexte, une pente d'association initiale différente sera observée en fonction du flux et la comparaison des vitesses d'association en fonction du flux utilisé permet de calculer la concentration active de l'analyte. Ainsi, l'échantillon est injecté sur la puce à deux flux différents, un flux lent et un flux rapide, ces deux flux étant choisis de sorte à obtenir des pentes d'association initiale différentes. Les conditions de température, de concentration de l'échantillon et de temps d'injection restent les mêmes.

La puce est régénérée entre deux mesures de sensorgramme. Par régénération est entendu que la puce retrouve son état initial dans lequel la surface ne porte que l'agent de capture. Les solutions de régénération sont bien connues de l'homme du métier, par exemple des solutions acides, notamment à pH inférieur à 3.

Les flux lents et rapides sont choisis de sorte que la pente d'association initiale au flux lent soit suffisamment importante (supérieure à 0,3 RU/s à 5 μl/min) et que des sensorgrammes suffisamment différents entre les deux flux soient obtenus. Cette différence est indiquée par un « QC ratio » supérieur à 0,2. Le QC ratio est le quotient Q reflétant le degré de limitation de transport de masse :

$$Q = \frac{pente\ d'association\ initiale\ au\ flux\ rapide}{pente\ d'association\ initiale\ au\ flux\ lent} \times \sqrt[3]{\frac{vitesse\ du\ flux\ lent}{vitesse\ du\ flux\ rapide}}$$

En particulier, le flux rapide est au minimum 5, 10, 15, 20 ou 50 fois plus rapide que le flux lent. Notamment, le flux rapide peut être 5, 10, 15, 20 ou 50 fois plus rapide que le flux lent. Par exemple, le flux lent peut être de 2-20 μl/min, de préférence de 2-10 μl/min, et de manière particulièrement préférée de 5 μl/min. Le flux rapide peut être de 10-100 μl/min, de préférence de 25-100 μl/min, et de manière particulièrement préférée de 100 μl/min. Le temps d'injection peut par exemple être de 30 à 100 s, de préférence de 40 à 60s, et de manière préférée de 50 s.

Pour la détermination de la concentration active, la capture de la plus grande quantité possible de ligand est recherchée.

De préférence, dans le contexte d'un analyte étant un anticorps anti-HLA, pour la détermination de la concentration active, l'antigène HLA est capturé par l'agent de capture en grande quantité. Par grande quantité est entendu que la quantité de l'antigène HLA capturé est d'au minimum 100 RU, de préférence de plus de 200, 300 ou 400 RU. Dans un mode de réalisation préférée, la quantité de l'antigène HLA immobilisée est de 500 à 8000 RU. De préférence, la quantité de l'antigène HLA immobilisée est de 500 à 2000 RU.

Les auto-blancs avec les ligands témoins et les tests avec les ligands cibles sont réalisés dans les mêmes conditions, excepté la différence entre les ligands.

Les sensorgrammes obtenus avec les auto-blancs sont soustraits des sensorgrammes obtenus avec les ligands cibles. La concentration active de l'analyte testé peut ainsi être calculée.

Détermination des constantes cinétiques d'interaction entre l'analyte et le ligand

La SPR permet également de déterminer l'affinité du complexe anticorps anti-HLA-antigène HLA en injectant des concentrations croissantes d'analyte (et donc d'échantillon) sur la surface. Les expériences peuvent être réalisées en plusieurs cycles d'association - dissociation puis régénération (analyse cinétique de cycles multiples ou *multiple cycle kinetics analysis* (MCK)), ou en injectant des concentrations croissantes de l'analyte jusqu'à saturation de la cible, sans cycle de régénération entre les injections (analyse cinétique de cycle simple ou *single cycle kinetics analysis* (SCK)).

De préférence, pour la détermination des constantes cinétiques d'interaction, le ligand est capturé par l'agent de capture en faible quantité, en particulier la plus faible quantité possible. Dans le contexte d'un analyte qui est un anticorps anti-HLA, par faible quantité est entendu que la quantité de l'antigène HLA immobilisée est d'au maximum 150 RU, de préférence de moins de 100 RU. Dans un mode de réalisation préférée, la quantité de l'antigène HLA immobilisé est de 20 à 100 RU, de préférence de 50 à 100 RU.

Dans ce contexte, la même température, le même flux et le même temps d'injection sont utilisés pour des concentrations variables de l'analyte à tester, et donc des concentrations/dilutions différentes de l'échantillon biologique. Au minimum, l'échantillon est injecté à deux concentrations différentes, par exemple, à 2, 3, 4, 5 ou 6 concentrations. Dans un mode de réalisation préférée, 3 concentrations différentes sont utilisées. De préférence, lorsque la méthode SCK est mise en œuvre, les concentrations différentes sont croissantes. Les concentrations différentes peuvent varier d'un facteur 2 à 10, par exemple d'un facteur 2, 3, 4, ou 5.

La vitesse d'injection ou flux peut par exemple être de 1-100 μl/min, de préférence de 10-50 μl/min, et de manière particulièrement préférée de 25 μl/min.

Le temps d'injection peut par exemple être de 10 à 100 s, de préférence de 40 à 70s, et de manière préférée de 60 s.

Les auto-blancs et les tests avec les ligands cibles sont réalisés dans les mêmes conditions, excepté la différence de ligands.

Les sensorgrammes obtenus avec les auto-blancs sont soustraits des sensorgrammes obtenus avec les ligands cibles.

Les logiciels d'analyse permettent de réaliser un ajustement ou « fit » des sensorgrammes suivant un modèle d'interaction donné, le plus souvent un modèle bimoléculaire simple 1:1. Les constantes élémentaires d'association et de dissociation, $k_a$ et $k_d$, respectivement, sont déterminées directement à partir de cette analyse.

Kit

Décrit ici est aussi un un kit adapté pour réaliser la méthode selon la présente invention.

En particulier, le kit comprendra une puce de SPR présentant plusieurs pistes différentes.

Dans un mode de réalisation, dans le contexte d'un couple analyte/ligand, la puce pourra comprendre une ou plusieurs pistes choisies sur laquelle ou lesquelles sera immobilisé un agent de capture spécifique du ligand comme détaillé ci-dessus.

Dans un mode de réalisation particulier, dans le contexte d'un analyte qui est un anticorps anti-HLA, la puce pourra comprendre une ou plusieurs pistes choisies parmi les pistes suivantes :

- une piste sur laquelle sera immobilisé un agent de capture spécifique des antigènes HLA de classe I, par exemple spécifique de la β2 microglobuline,
- une piste sur laquelle sera immobilisé un agent de capture spécifique des antigènes HLA-DQ (classe II) ;
- une piste sur laquelle sera immobilisé un agent de capture spécifique des antigènes HLA-DR (classe II) ; et
- une piste sur laquelle sera immobilisé un agent de capture spécifique des antigènes HLA-DP (classe II).

De préférence, la puce comprend au moins deux ou trois pistes choisies parmi les pistes ci-dessus mentionnées.

Dans un mode de réalisation préféré, la puce comprend :

- une piste sur laquelle sera immobilisé un agent de capture spécifique des antigènes HLA de classe I, par exemple spécifique de la β2 microglobuline ;
- une piste sur laquelle sera immobilisé un agent de capture spécifique des antigènes HLA-DQ (classe II) ; et
- une piste sur laquelle sera immobilisé un agent de capture spécifique des antigènes HLA-DR (classe II) ;

Dans un mode de réalisation, la puce de SPR comprendra en outre une piste de référence.

Le kit pourra comprendre en outre un ou plusieurs antigènes HLA utiles pour réaliser les auto-blancs et/ou correspondre aux HLA cibles des anticorps anti-HLA pour réaliser les tests.

Le kit pourra comprendre en outre un ou plusieurs anticorps anti-HLA bien caractérisés qui pourraient servir de contrôle.

Le kit peut comprendre en outre des réactifs nécessaires à la réalisation de la présente méthode, par exemple sélectionnés parmi le tampon de charge, du DTT, une résine de protéine G, etc... Le kit peut également comprendre une notice d'utilisation.

Un kit tel que défini ci-dessus peut être utilisé pour la détermination de la concentration active d'un analyte et/ou des constantes cinétiques d'association de l'analyte au ligand dans un échantillon biologique complexe, en particulier pour la détermination de la concentration active et/ou des constantes cinétiques d'association d'un anticorps anti-HLA avec l'antigène HLA reconnu par l'anticorps compris dans un échantillon biologique complexe.

Utilisation de la méthode

L'invention est destinée à des besoins cliniques et de recherche.

Notamment, la concentration active des anticorps anti-HLA contenus dans des milieux complexes et leurs constantes cinétiques d'interaction avec leur cible sont parfaitement inconnues, alors que ces paramètres pourraient avoir une importance cruciale dans la détermination de leur caractère pathologique, notamment dans le cadre des transplantations. Il faut noter que la détermination des constantes cinétiques d'interaction nécessite de connaître la concentration active, et que la concentration active est en soi un paramètre pouvant être utile de manière indépendante des constantes cinétiques.

En clinique, la détermination de ces paramètres pourrait permettre de déterminer les patients les plus à risque de perdre leur greffon en raison de la présence d'anticorps anti-HLA (intérêt diagnostique et pronostique), mais également de suivre l'efficacité d'un traitement (modification de la concentration des anticorps et/ou de leur affinité). Après l'accumulation d'un grand nombre de données cliniques et biologiques, il serait peut-être possible d'améliorer les stratégies d'appariement des couples donneurs/receveurs en choisissant des antigènes HLA plus « compatibles » entre eux, c'est-à-dire induisant la production d'anticorps anti-HLA peu concentrés ou de faible affinité.

En recherche, il serait possible d'utiliser des anticorps anti-HLA caractérisés de manière précise, en utilisant la présente invention, sur des modèles *in vitro* couramment employés dans le domaine (cellules endothéliales, lymphocytes du sang périphérique, etc...), et d'étudier l'impact de la concentration active et des constantes cinétiques sur les mécanismes de pathogénicité de ces anticorps : activation du complément, activation cellulaire, cytotoxicité cellulaire dépendante des anticorps. L'invention permettrait également de mieux comprendre les résultats donnés par les techniques de routine que sont le Single Antigen en format Luminex® et les crossmatches par microlymphocytotoxicité et cytométrie en flux.

La méthode développée par les inventeurs présente un intérêt dans d'autres domaines d'application et, de manière non exhaustive :

- pour la mesure de la concentration de marqueurs tumoraux dans des milieux complexes tels que les échantillons humains, par exemple la bêta-2-microglobuline (B2M), ou l'antigène prostatique (PSA). Dans ce contexte, l'agent de capture peut par exemple être un anticorps anti-anticorps, le ligand cible est un anticorps spécifique du marqueur tumoral, le ligand témoin est un anticorps ne présentant pas d'affinité pour ce marqueur.

- pour le dosage de $\beta 2$ microglobuline. En effet, la $\beta 2$ microglobuline est une protéine de 11800 Da qui est dosée quotidiennement dans les laboratoires en tant que marqueur de première intention dans le myélome multiple et les lymphopathies B malignes. Son dosage est aussi utilisé dans le pronostic et la surveillance thérapeutique des infections à VIH, dans le suivi de maladies inflammatoires chroniques, dans l'exploration et le suivi de la fonction rénale, chez les patients hémodialysés ou ayant bénéficié d'une transplantation rénale. Ses valeurs de référence sont de l'ordre de 1 mg/ml (un peu plus de 80 nM). Dans ce contexte, l'agent de capture pourrait être un anticorps anti-anticorps, le ligand témoin serait un anticorps quelconque n'ayant pas d'affinité pour la $\beta 2$ microglobuline, et le ligand cible serait un anticorps anti -$\beta 2$ microglobuline.

- pour la mesure de la concentration active d'anticorps dirigés contres des antigènes vaccinaux et éventuellement la définition de leurs paramètres cinétiques d'interaction avec leur cible, afin d'évaluer l'efficacité d'un vaccin par exemple. Dans ce contexte, l'agent de capture pourrait être un anticorps dirigé contre l'antigène ou une étiquette attachée à l'antigène, le ligand témoin serait une molécule différente des antigènes mais de masse similaire, et le ligand cible serait un ou des antigènes du vaccin. De la même manière, une application possible concerne tout anticorps produit suite à une infection, afin de connaître l'efficacité de la réponse d'un individu et son niveau de protection vis-à-vis du pathogène considéré.

- pour la mesure de la concentration active de cytokines et éventuellement la définition de leurs paramètres cinétiques d'interaction avec leur récepteur. Dans ce contexte, l'agent de capture pourrait être un anticorps dirigé contre le récepteur ou une étiquette attachée au récepteur, le ligand témoin serait un récepteur ou une molécule n'ayant pas d'affinité pour la cytokine, et le ligand cible serait le récepteur de la cytokine. Ceci permettrait de déterminer la fraction de la cytokine capable d'interagir avec son récepteur, sachant que certaines cytokines peuvent être complexées à des récepteurs solubles, ce qui les rend inactives.

- pour la mesure de la concentration active de molécules pharmacologiques et éventuellement la définition de leurs paramètres cinétiques d'interaction avec leur cible, par exemple en capturant leur cible à l'aide d'un anticorps anti-cible ou anti-tag.

- pour la mesure de la concentration active d'anticorps anti-médicaments et éventuellement la définition de leurs paramètres cinétiques d'interaction avec leur cible médicamenteuse. Par exemple, immobilisation de la cible d'une biothérapie (ex : TNF$\alpha$), puis capture de la biothérapie (anti-TNF$\alpha$), et injection de l'échantillon humain sur la surface. L'auto-blanc pourrait être obtenu en utilisant une autre biothérapie dirigée contre le TNF$\alpha$.

## Description des Figures

Figure 1. Etapes successives conduisant au résultat escompté à partir du sérum de patient.

Figure 2. CFCA auto-blanc à partir d'un échantillon en provenance du sérum d'un patient ayant des anticorps anti-DQ2 (auto-blanc DQ5). Dilution de l'échantillon au 1/200, concentration mesurée 1,4 nM, concentration dans l'échantillon 270 nM, concentration dans le sérum après correction en fonction des dosages IgG 460 nM, QC ratio 0,496.

Figure 3. SCK auto-blanc à partir d'un échantillon en provenance du sérum d'un patient ayant des anticorps anti-DQ2 (auto-blanc DQ5). Concentration utilisée, déduite de la CFCA auto blanc : 2,16 nM, 10,8 nM et 54 nM. $k_a$ = 1,493.10$^6$ M$^{-1}$.s$^{-1}$, $k_d$ = 2,482.10$^{-4}$ s$^{-1}$, $K_D$ = 1,663.10$^{-10}$ M.

Figure 4. Comparaison du blanc (sensorgrammes clairs) et de la réaction (sensorgrammes sombres) d'une SCK auto-blanc à partir d'un échantillon en provenance du sérum d'un patient ayant des anticorps anti-DQ2 (auto-blanc DQ5).

Figure 5. Système permettant de mesurer la concentration de la B2m par SPR CFCA dans des milieux complexes.

Figure 6. Sensorgrammes de CFCA pour le dosage de la B2m dans les différentes conditions : blanc classique (PBS-T) et auto-blanc dans un sérum traité au DTT et dialysé dilué au 1/1000, 1/100 et 1/10. Les sensorgrammes sont en gris, l'ajustement réalisé par le logiciel d'analyse en noir.

## Exemples

L'objet de l'invention est l'aboutissement de deux années d'expériences destinées à pouvoir analyser des milieux complexes tels que des échantillons de patients, donc à éliminer le NSB qu'ils présentent systématiquement.

Les inventeurs ont tout d'abord testé des sérums de patients dilués au 1/10ème sur la surface où le B2M-01 (IgG2a de souris anti-B2M) était immobilisé (16000 RU environ). Tous les sérums montraient une forte interaction avec la surface (Tableau 1), qu'il y ait ou non immobilisation de l'anticorps de capture.

**Tableau 1.** Observation du NSB avec les sérums de patients (dilution 1/10 dans PBS-T) sur la piste de référence (piste 3 vide) et la piste avec 16000 RU de B2M-01 immobilisés. Valeurs en RU. Flux 25$\mu$l/min, durée d'injection = 1 min.

| Injection | Piste 3 (vide) | Piste 4 (B2M-01) | 4-3 |
|---|---|---|---|
| Sérum 001 | 195 | **1871** | **1650** |
| Sérum 003 | 59 | **2000** | **1900** |
| Sérum 004 | 44 | **1544** | **1513** |
| Sérum 005 | 61 | **1260** | **1195** |
| Sérum 007 | 208 | **1988** | **1779** |

(suite)

| Injection | Piste 3 (vide) | Piste 4 (B2M-01) | 4-3 |
|---|---|---|---|
| Sérum 010 | 87 | **1412** | **1300** |

Divers traitements ont été appliqués aux échantillons pour tenter de diminuer le NSB (élimination des petites protéines par dialyse ou inactivation du complément). Ces traitements se sont avérés être seulement partiellement efficaces car une fixation résiduelle non spécifique persistait, à des degrés divers. Le NSB résiduel aurait pu ne pas poser de problème si celui-ci avait été identique pour tous les patients. Dans ce cas, un sérum ou un mélange de sérums dépourvus d'anti-HLA aurait pu être utilisé comme blanc. Cependant, il a été observé que le NSB résiduel était très variable parmi les échantillons.

Les inventeurs ont alors entrepris de purifier les IgG sériques sur résines de protéine G. Les premiers résultats, utilisant les IgG purifiées diluées dans du PBS-T, n'étaient pas convaincants. Ils ont même observé plus de NSB avec les purifiats sur protéine G et que le NSB était également variable sur la piste de référence, en fonction de l'échantillon analysé.

Un certain nombre d'autres essais a été réalisé : dénaturation du sérum à la chaleur, épuisement du NSB par le passage de l'échantillon sur des pistes en amont de la piste test avec anti-B2M immobilisé, pré-saturation de la piste avec un sérum pur, ajout de BSA au tampon de course, désactivation de la surface, immobilisation de l'ancre sur la piste de référence. Tous se sont également avérés être insuffisamment efficaces.

A ce stade des investigations, les inventeurs avaient intégré qu'il était impossible de réduire à zéro le NSB obtenu lorsque des échantillons en provenance de patients étaient utilisés. Néanmoins, une information cruciale obtenue était que la capture du HLA diminuait le NSB, certainement parce que la masse de protéines présente sur la puce était augmentée en comparaison à l'absence de capture.

C'est ainsi que les inventeurs ont eu l'idée d'utiliser chaque échantillon comme son propre blanc sur une piste sur laquelle une molécule HLA non reconnue par les anticorps présents dans l'échantillon est capturée en quantité équivalente à la molécule HLA cible de ces anticorps.

Les inventeurs ont tout d'abord validé cette méthode à l'aide d'anticorps monoclonaux, utilisés dans des dérivés de sérum de patients non immunisés anti-HLA, pour lesquels ils ont déterminé la concentration active par CFCA. Ceci a été réalisé pour un anticorps anti-HLA de classe I monoclonal de souris (anti-HLA-A2) (capture des molécules HLA par un anticorps anti-B2m, clone B2M-01) et un anticorps anti-HLA de classe II monoclonal de souris (anti-DQ2) (capture des molécules HLA par un anticorps anti-DQ commercial, clone Tu169). La partie du procédé se déroulant sur l'appareil de SPR a été la suivante :

1 - Immobilisation de l'anticorps de capture par chimie de couplage sur la puce de SPR;
2 - Réalisation de la méthode CFCA :

- Pour le blanc, dans un premier temps, un antigène HLA non reconnu par l'anticorps à tester est capturé en grande quantité sur la surface, puis la solution contenant l'anticorps à tester est injectée à un flux déterminé pendant un temps déterminé. Enfin, après un court temps de dissociation, la surface est régénérée, ce qui permet de passer à un cycle suivant.
- Pour le test lui-même, dans un premier temps, l'antigène HLA reconnu par l'anticorps à tester est capturé en grande quantité sur la surface, puis la solution contenant l'anticorps à tester est injectée à un même flux déterminé pendant un même temps déterminé. Enfin, après un court temps de dissociation, la surface est régénérée, ce qui permet de passer au cycle suivant.
- Le logiciel d'analyse de l'appareil réalise la correction en soustrayant le sensorgramme du blanc à celui du test, c'est ce que l'on appelle le double-référencement (*double-referencing*).

Les inventeurs ont pu tester l'auto-blanc dans quatre conditions différentes pour chaque classe d'HLA en comparaison au tampon de course (RB pour running buffer qui est du PBS-T). Pour cela, ils ont utilisé des milieux complexes dérivés de sérums de patients dont les caractéristiques de NSB sont les suivantes (dilution ½ dans PBS-T avec 10% de « NSB *reducer* », une solution de dextran fournie par GE Healthcare qui n'est cependant pas indispensable) (Tableau 2) :

**Tableau 2.** NSB des échantillons utilisés pour valider la méthode auto-blanc pour la CFCA. Flux 25µl/min, durée d'injection = 1 min

| Ancre | B2M-01 | | Tu169 (anti-DQ) | |
|---|---|---|---|---|
| Traitement du sérum | Chaleur + protéine G | Chaleur + DTT + dialyse | Chaleur + protéine G | Chaleur + DTT + dialyse |
| BOY | 20 | 341 | 24 | 29 |
| MOU | 28 | 235 | 49 | 75 |
| PIC | 23/17 | 151/120 | 24 | 32 |
| TAS | 30/25 | 423/349 | 22 | 45 |

Ce tableau souligne l'hétérogénéité du NSB en fonction des échantillons et des prétraitements mais également de l'ancre utilisée. Les tests suivants se sont appliqués à utiliser des échantillons de NSB différents auxquels a été comparée la méthode réalisée en PBS-T (Tableau 3).

**Tableau 3.** CFCA de deux anticorps monoclonaux dans des milieux complexes en utilisant la méthode auto-blanc, en comparaison avec la méthode en milieu simple « RB » qui définit la cible de concentration. La molécule HLA d'auto-blanc était le HLA-A11 pour l'anti-HLA-A2, et HLA-DQ7 pour l'anti-HLA-DQ2. Le QC ratio est un indice de qualité qui témoigne de la présence d'un transport de masse. Les résultats sont fiables à 100% si le QC ratio est supérieur à 0,2.

| | | Classe I (A20L - A2/A11) | | | DQ (DQ20L - DQ2/DQ7) | | |
|---|---|---|---|---|---|---|---|
| | Echantillon | PIC | TAS | RB (cible) | BOY | MOU | RB (cible) |
| Chaleur + protéine G | Concentration mesurée (nM) | 1,5 | 1,7 | 1,4 | 0,69 | 0,65 | 0,73 |
| | QC ratio | 0,549 | 0,509 | 0,558 | 0,446 | 0,464 | 0,399 |
| Chaleur + DTT + dialyse | Concentration mesurée (nM) | 0,96 | 1,8 | 1,4 | 0,96 | 0,91 | 1,3 |
| | QC ratio | 0,952 | 1 | 0,558 | 0,391 | 0,405 | 0,296 |

Ce tableau met en évidence la fiabilité des résultats obtenus grâce à l'auto-blanc, la différence avec la cible étant de l'ordre du dixième de nM.

Les inventeurs ont alors essayé cette approche sur 5 patients ayant des anticorps anti-HLA DQ2 et DQ7 dont les sérums avaient été traités à la chaleur, DTT, puis dialysés. Les résultats sont présentés dans le Tableau 4 :

**Tableau 4.** CFCA d'anticorps anti-HLA DQ à partir d'échantillons en provenance de sérums de patients. Ces sérums avaient été identifiés comme contenant des anticorps anti-HLA DQ2 ou DQ7 par la technique Single Antigen Luminex® (SAFB) dont la valeur de fluorescence (MFI) est précisée dans le tableau. Le traitement appliqué aux sérums était un traitement à la chaleur (56°C 30 min), DTT (5mM 37°C 30 min) puis dialyse contre du PBS-T (membrane 100 kDa, 1 heure, 6 heures puis sur la nuit). Le dosage des IgG totales réalisé avant et après traitement du sérum permet de déterminer la concentration initiale des IgG anti-HLA présentes dans le sérum. Pour passage sur l'appareil de SPR, les échantillons ont été utilisés dilués au ½ dans du PBS-T avec 10% de « NSB *reducer* ». Pour les anti-DQ2 (DQB1*02:01/DQA1*05:01), l'auto-blanc était réalisé sur du HLA-DQ7 capturé, et inversement pour les anti-DQ7.

| | Cible | SAFB MFI | Concentration (nM) | Concentration corrigée (nM) | IgG initiale (g/L) | IgG finale (g/L) |
|---|---|---|---|---|---|---|
| BEL 1/2 | DQ2 | 11237 | 1.6 | 2,18 | 9.65 | 7.07 |
| BRI 1/2 | DQ2 | 20507 | 7.6 | 12,71 | 11.00 | 6.58 |
| LAP 1/2 | DQ2 | 15000 | 3.3 | 4,90 | 9.02 | 6.08 |
| PLA 1/2 | DQ7 | 20627 | 3.4 | 5,46 | 8.33 | 5.19 |
| THI 1/2 | DQ7 | 21464 | 2.1 | 2,83 | 11.60 | 8.61 |

Il est intéressant de noter que les inventeurs n'ont pas observé de relation parfaite entre la concentration active des anti-HLA-DQ obtenus par SPR et la MFI en SAFB. Ceci suggère que les données obtenues par SPR pourraient apporter des informations différentes. Ceci pourrait être crucial pour les patients.

A noter qu'il existe de forts « sauts de tampons » lors de l'utilisation de sérum traité chaleur+DTT+dialyse, et que le temps nécessaire à l'obtention d'un échantillon prêt à être utilisé sur la plateforme Biacore est de 24 heures (après identification des anticorps en SAFB qui dure une demi-journée). Les inventeurs ont plus récemment opté pour le deuxième pré-traitement évoqué, c'est-à-dire un traitement à la chaleur de 1 ml de sérum (56°C pendant 30 min) puis une purification sur résine de protéine G (1 heure), et enfin une concentration du purifiat pour revenir à un volume équivalent (1 à 1,2 ml) dans du PBS-T. Le dosage des IgG totales avant et après purification permet de revenir à la concentration initiale des anticorps anti-HLA.

Il est important de noter que ces deux traitements permettent de s'affranchir des anti-HLA d'isotype IgM pouvant être présents dans le sérum des patients en même temps que les IgG. Ces IgM ont été identifiées comme de potentielles molécules interférentes en SAFB, mais auraient pu également gêner l'analyse des anti-HLA d'isotype IgG par SPR.

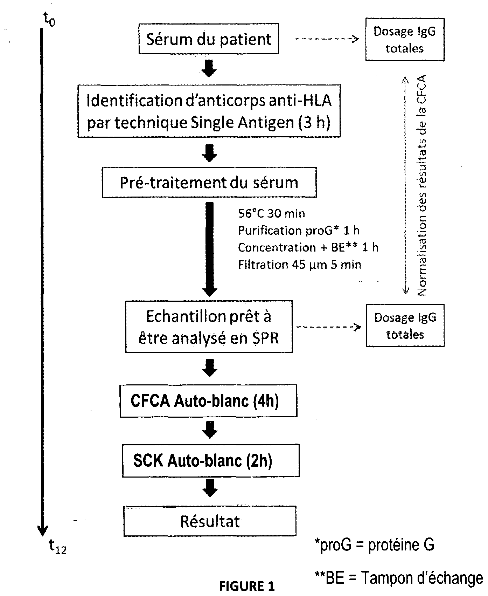

La Figure 1 détaille les étapes successives conduisant au résultat escompté à partir du sérum de patient. Le flux de travail présenté en Figure 1 montre que le temps de réalisation de l'examen est tout à fait compatible avec une application en clinique, la caractérisation des anticorps anti-HLA n'étant pas un examen urgent.

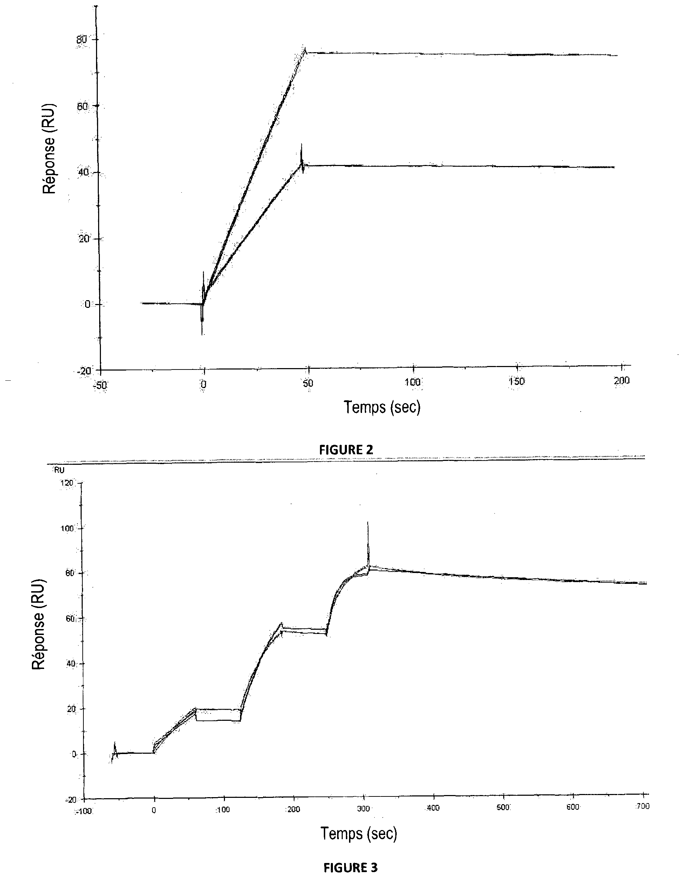

Au final, il est donc tout à fait possible d'obtenir un résultat en deux jours de travail. Ci-dessous est présenté l'exemple d'un patient ayant des anticorps anti-DQ2 ayant été étudié par ce procédé. Les Figures 2 et 3 présentent les résultats de la CFCA puis de la SCK auto blanc. L'ajustement des sensorgrammes au modèle cinétique d'interaction est très satisfaisant comme le montre la superposition des courbes expérimentales avec les courbes théoriques.

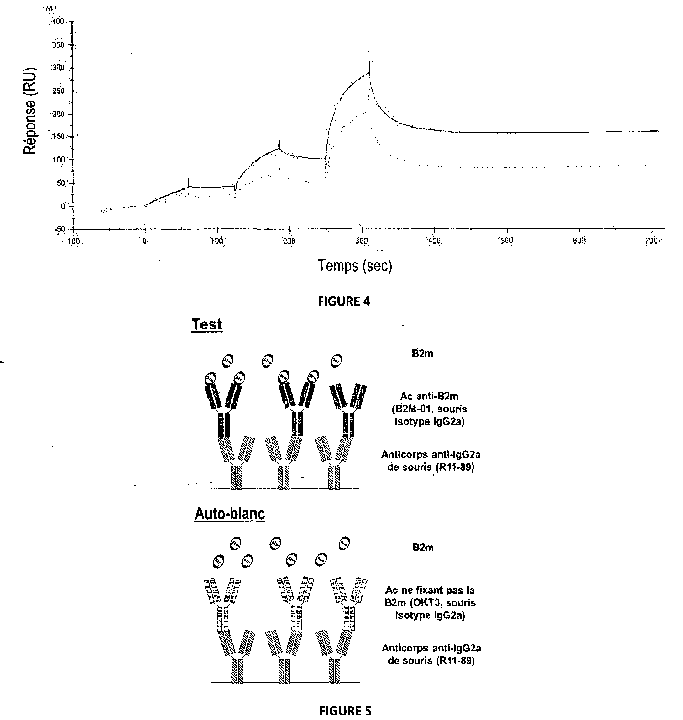

La figure 4 met en évidence le niveau de NSB lors de l'injection d'échantillon en concentration croissante et l'importance de l'utilisation de l'autoblanc permettant de corriger ce NSB. En effet, il apparait clairement une forte fixation sur la piste en l'absence de la molécule HLA cible.

La méthode est parfaitement reproductible (Tableaux 5 et 6).

**Tableau 5.** CFCA auto-blanc à partir d'un échantillon en provenance du sérum d'un patient ayant des anticorps anti-DQ2 (auto-blanc DQ5). Dilution de l'échantillon au 1/200 ou 1/100 à partir d'aliquots congelés testés à des jours différents.

|  | Dilution | Concentration mesurée (nM) | Concentration échantillon (nM) | QC ratio |
|---|---|---|---|---|
| Aliquot 1 | 200 | 1,4 | 270 | 0,496 |
| Aliquot 2 | 200 | 1,5 | 290 | 0,526 |
| Aliquot 3 | 100 | 3,2 | 320 | 0,439 |

**Tableau 6.** SCK auto-blanc à partir d'un échantillon en provenance du sérum d'un patient ayant des anticorps anti-DQ2 (auto-blanc DQ5). Tests réalisés à partir d'aliquots congelés à des jours différents.

|  | $k_a$ (1/Ms) | $k_d$ (1/s) | $K_D$ (M) |
|---|---|---|---|
| Aliquot 1 | $1,49.10^6$ | $2,48.10^{-4}$ | $1,66.10^{-10}$ |
| Aliquot 2 | $1,36.10^6$ | $2,37.10^{-4}$ | $1,74.10^{-10}$ |
| Aliquot 3 | $1,24.10^6$ | $2,38.10^{-4}$ | $1,90.10^{-10}$ |

Les inventeurs ont répété l'utilisation de cette approche sur 10 patients supplémentaires ayant des anticorps anti-HLA DQ2, DQ4, DQ6, DQ7, DQ9 et ayant bénéficié de différents types de transplantation (poumons, cœur, rein, foie). Les résultats sont présentés dans le Tableau 7.

**Tableau 7.** CFCA et SCK d'anticorps anti-HLA DQ à partir d'échantillons en provenance de sérums de patients. Ces sérums avaient été identifiés comme contenant des anticorps anti-HLA DQ par la technique Single Antigen Luminex® (SAFB) dont la valeur de fluorescence (MFI) est précisée dans le tableau (chaîne alpha associée à la chaîne beta précisée entre parenthèses lorsque pertinent).

| HLA-DQ Cible | HLA-DQ Blanc | SAFB MFI | Concentration (nM) | Concentration corrigée (nM) | IgG initiale (g/L) | IgG finale (g/L) | $k_a$ (1/Ms) | kd (1/s) | $K_D$(M) |
|---|---|---|---|---|---|---|---|---|---|
| DQ2(05) | DQ5 | 15570 | 5,2 | 7,85 | 10,4 | 6,90 | $5,71.10^6$ | $5.59.10^{-4}$ | $9,79.10^{-11}$ |
| DQ2(05) | DQ5 | 13802 | 15 | 24,15 | 11,3 | 7,02 | $2,43.10^6$ | $1.37.10^{-4}$ | $5,61.10^{-11}$ |
| DQ7 | DQ5 | 20442 | 65 | 100,6 | 6,47 | 4,18 | $1,19.10^6$ | $1.76.10^{-4}$ | $1.50.10^{-10}$ |
| DQ7 | DQ5 | 18172 | 14 | 22,3 | 13,2 | 8,3 | $2,13.10^6$ | $1,80.10^{-3}$ | $8.59.10^{-10}$ |
| DQ7 | DQ5 | 20763 | 11 | 17,8 | 11,7 | 7,23 | $5,38.10^5$ | $6,83.10^{-4}$ | $1.27.10^{-9}$ |
| DQ2(05) | DQ5 | 23076 | 280 | 459,2 | 13,4 | 8,17 | $8.15.10^5$ | $2.35.10^{-4}$ | $2.88.10^{-10}$ |
| DQ2(02) | DQ6 | 18807 | 9,7 | 15,8 | 12,1 | 7,41 | $3,15.10^6$ | $1,4.10^{-3}$ | $4.58.10^{-10}$ |
| DQ4 | DQ5 | 18401 | 27,0 | 40,0 | 7,49 | 5,06 | $1,72.10^6$ | $4,29.10^{-4}$ | $2.49.10^{-10}$ |
| DQ6 | DQ5 | 21658 | 8,6 | 39,8 | 10,5 | 2,27 | $1,97.10^6$ | $5.81.10^{-4}$ | $2.95.10^{-10}$ |
| DQ9 | DQ6 | 20000 | 14,0 | 21,3 | 4,29 | 2,82 | $1,52.10^6$ | $5,93.10^{-4}$ | $3.89.10^{-10}$ |

Les inventeurs ont également appliqué cette approche sur un système autre que les anticorps anti-HLA, à savoir le dosage de la bêta-2 microglobuline (B2m) dans des milieux complexes. La mesure des constantes cinétiques d'interaction de la B2m pour son anticorps correspondant n'a pas été réalisée car ne possède aucune signification clinique ou biologique.

La partie du procédé se déroulant sur l'appareil de SPR a été la suivante (Figure 5) :

1 - Immobilisation de l'anticorps de capture par chimie de couplage sur la puce de SPR (anti-IgG2a de souris, clone R11-89, BD Biosciences) ;

2 - Réalisation de la méthode CFCA :
Pour le blanc, dans un premier temps, un anticorps ne reconnaissant pas la B2m (clone OKT3, anticorps anti-CD3, BD Biosciences) a été capturé en grande quantité sur la surface, puis la solution à tester contenant la B2m a été injectée à un flux déterminé pendant un temps déterminé. Enfin, après un court temps de dissociation, la surface a été régénérée, ce qui permettait de passer à un cycle suivant.

Pour le test lui-même, dans un premier temps, un anticorps reconnaissant la B2m (clone B2M-01) a été capturé en grande quantité sur la surface, puis la solution contenant la B2m a été injectée à un même flux déterminé pendant un même temps déterminé. Enfin, après un court temps de dissociation, la surface a été régénérée, ce qui permet de passer au cycle suivant.

Le logiciel d'analyse de l'appareil a réalisé la correction en soustrayant le sensorgramme du blanc à celui du test, c'est ce que l'on appelle le double-référencement (double-referencing).

La concentration d'une solution de B2m recombinante (Thermo Fisher) diluée au 1/10 000 dans du tampon de course (PBS-T) a tout d'abord été mesurée par CFCA capture, en utilisant comme blanc du tampon de course. La concentration mesurée était de 4,3 nM.

La CFCA capture a ensuite été réalisée en auto-blanc dans un milieu complexe (sérum de sujet sain traité à la chaleur, au DTT, puis dialysé contre du PBS-T sur membrane de 100 kDa) utilisé à 3 dilutions différentes et donnant ainsi lieu à différents niveaux de NSB. Les valeurs de NSB relevées sur l'anticorps OKT3 ont été les suivantes (tableau 8).

**Tableau 8.** Quantité de NSB généré par les milieux complexes utilisés pour la validation de l'auto-blanc en milieux complexes pour le dosage de la B2m.

| Milieu complexe | NSB au flux 5 $\mu$l/min (RU) | NSB au flux 100 $\mu$l/min (RU) |
|---|---|---|
| Sérum 1/10 | 342 | 575 |
| Sérum 1/100 | 71 | 152 |
| Sérum 1/1000 | 14 | 26 |

Les concentrations de B2m mesurées dans ces échantillons ont été les suivantes (tableau 9).

**Tableau 9.** Dosage de la B2m par CFCA capture auto-blanc au sein de différents milieux complexes.

| Milieu | Concentration mesurée (nM) |
|---|---|
| PBS-T | 4,3 |
| Sérum 1/1000 | 4,0 |
| Sérum 1/100 | 4,0 |
| Sérum 1/10 | 4,7 |

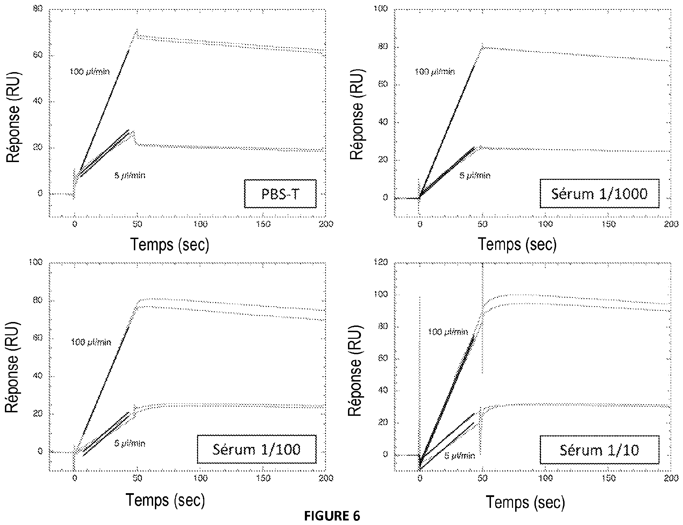

Les sensorgrammes des CFCA sont représentés dans la Figure 6.

Matériels et Méthodes

• *Anticorps et molécules HLA*

Les anticorps anti-HLA de classe I monoclonaux, d'origine murine, utilisés étaient : les anticorps pan-classe I W6/32 et anti-beta2-microglobuline (clone B2M-01) (ThermoFisher Scientific, Rockford, IL) et un anticorps anti-HLA-A2 (One Lambda, Inc, Canoga Park, CA). Les anticorps anti-HLA de classe II monoclonaux, d'origine murine, utilisés étaient : l'anticorps pan-DQ (clone Tu169) (BD Biosciences, Le Pont de Claix, France) et un anticorps anti-HLA-DQ2 (One Lambda, Inc, Canoga Park, CA). Les molécules HLA purifiées HLA-A*02:01 (A2), A*11:01 (A11), DQB1*02:01/DQA1*05:01 [DQ2(05)], DQB1*02:01/DQA1*02:01 [DQ2(02)], DQB1*03:01/DQA1*05:05 (DQ7), DQB1*03:03/DQA1*02:01 (DQ9), DQB1*04:01/DQA1*03:03 (DQ4), DQB1*05:01/DQA1*01:01 (DQ5) et DQB1*06:03/DQA1*01:03 (DQ6) étaient produites par One Lambda. Un anticorps anti-CD3 (clone OKT3, BD Biosciences) et anti-IgG2a de souris (clone R11-89, BD Biosciences) ont été utilisés.

• *Expériences en résonance plasmonique de surface (SPR)*

Les expériences de SPR ont été réalisées à 25°C sur un Biacore™ T200 (GE Healthcare Life Sciences, Uppsala, Sweden) avec des biopuces CM5 (Biacore™). Les sensorgrammes ont été analysés avec le logiciel d'évaluation Biacore T200 Evaluation Software. Les anticorps de capture ont été immobilisés à l'aide d'une chimie de couplage par les amines, en utilisant un mélange de N-hydroxysuccinimide et N-ethyl-N'-dimethylaminopropyl carbodiimide selon les recommandations du fournisseur (GE Healthcare), après avoir été dilués dans une solution d'acétate de sodium (10 mM, pH 5), suivi par une désactivation de la surface par l'injection d'une solution d'éthanolamine (1 M, pH 8,5, GE Healthcare). Une piste laissée sans immobilisation a été utilisé pour réaliser une double correction des sensorgrammes. Les pics encore présents après cette étape n'ont pas été retirés car ils n'affectent pas les résultats. Sauf mention contraire, les échantillons ont été préparés dans du PBS-T 0,05% qui constituait le tampon de course. La régénération de la surface était réalisée par l'injection d'une solution de glycine (10 mM, pH 2,1, GE Healthcare) pendant 1 minute à 25µl/min. Pour les tests d'évaluation de la liaison non spécifique (NSB pour *non-specific binding),* les échantillons ont été injectés sur la surface pendant 1 min à 25 µl/min, sauf mention contraire.

• *Mesure de la concentration active*

Les expériences de « *calibration-free concentration analysis »* (CFCA) ont été réalisées après une capture préliminaire des ligands HLA (900 s à 2 µl/min pour la classe I, 840 s à 2 µl/min pour la classe II) qui étaient utilisés dans du tampon de course à une dilution permettant un haut niveau de capture, en quantité équivalente pour une même classe. Les anticorps anti-HLA étaient injectés pendant 50 s à 5 µl/min puis à 100 µl/min. Tous les échantillons et blancs ont été injectés en duplicats. Le coefficient de diffusion des anticorps anti-HLA a été calculé avec la formule suivante :

$$D = 342.3 \times 1/(\sqrt[3]{MW} \times f \times hrel) \times 10^{-11}$$

où MW est le poids moléculaire (150,000 Da), f est le coefficient de friction (1,2 pour les protéines globulaires), et hrel est la viscosité relative (0,89 à 25°C). Les critères de validation de la CFCA étaient ceux recommandés par Biacore™, c'est-à-dire une pente d'association initiale au flux lent suffisamment importante (supérieure à 0.3 RU/s à 5 µl/min) et des sensorgrammes suffisamment différents entre les deux flux, comme indiqué par un « QC ratio » fit supérieur à 0,2. Le QC ratio est le quotient Q reflétant le degré de limitation de transport de masse :

$$Q = \frac{pente\ d'association\ initiale\ au\ flux\ rapide}{pente\ d'association\ initiale\ au\ flux\ lent} \times \sqrt[3]{\frac{\text{vitesse du flux lent}}{\text{vitesse du flux rapide}}}$$

• *Mesure des paramètres cinétiques*

Les paramètres cinétiques ont été déterminés sur les mêmes pistes que pour les CFCA mais en capturant une quantité faible de HLA (moins de 100 RU) afin d'éviter les artéfacts cinétiques parfois observés avec de hautes densités de ligand. Les expériences ont été réalisées à 25 µl/min en utilisant la méthode « *single cycle kinetics »* (SCK) : trois concentrations croissantes de l'anticorps ont été injectées successivement sans régénération entre les injections. Tous les échantillons et blancs ont été injectés en duplicats. Les constantes d'association et de dissociation, $k_a$ et $k_d$, respectivement, ont été déterminées par un ajustement direct des sensorgrammes selon un modèle d'interaction de Langmuir 1:1. La constante d'équilibre de dissociation, $K_D$, a été calculée comme étant égale à $k_d/k_a$.

• *Sérums humains*

Les sérums humains utilisés venaient de sujets sains, de patients inscrits sur liste d'attente de transplantation d'organe ou de patients greffés. Un millilitre de ceux-ci a été utilisé sous différents états c'est-à-dire avec ou sans prétraitement, plusieurs prétraitements ayant pu être combinés. Le traitement à la chaleur consistait en l'incubation du sérum à 56°C pendant 30 min. Le traitement au dithiothréitol (DTT) consistait en l'ajout de DTT au sérum afin d'atteindre une concentration en DTT de 5 mM puis un chauffage à 37°C pendant 30 min. La purification des IgG sériques a été réalisée sur des billes de Sépharose couplées à de la protéine G (ThermoFisher Scientific) selon les recommandations du fournisseur. La concentration des purifiats a été réalisée à l'aide de dispositifs de centrifugation Amicon Ultra-4 10 kDa (ThermoFisher Scientific) à deux reprises afin de revenir à un volume de 1150 μl dans du tampon de course. La dialyse des échantillons a été réalisée contre le tampon de course à l'aide de dispositifs de dialyse Float-A-Lyzer G2 de cut-off 100 kDa (Spectrum Laboratories, Rancho Dominguez, CA). Tous les échantillons ont été filtrés sur des filtres de 0,45 μm avant utilisation. Le dosage des IgG totales a été effectué sur les sérums traités et non traités en immuno-néphélémétrie sur un automate BNII (Siemens Healthcare Diagnostics, Marburg, Germany).

• Tests SAFB

Les sérums ont été testés avec les kits Single Antigen Luminex® après traitement à l'EDTA (10 mM final) en suivant les recommandations du fournisseur (One Lambda) et ont été analysés sur un Luminex 100® (Luminex, Austin, TX). Les intensités de fluorescence (MFI pour mean fluorescence intensity) ont été normalisées avec la formule « baseline » (Logiciel Fusion®, One Lambda, Inc.).

*Matériels et méthodes pour le Tableau 2*

15000 RU d'anticorps ont été immobilisés pour la piste B2M-01 (classe I), et 18000 RU pour la piste Tu169 (DQ). Les sérums humains utilisés venaient de patients non immunisés anti-HLA inscrits sur liste d'attente de transplantation. Ceux-ci avaient subi un traitement à la chaleur puis une purification des IgG sur protéine G et concentration afin d'obtenir 1 ml de purifiât, ou bien un traitement à la chaleur suivi d'un traitement au DTT et une dialyse contre du tampon de course. Les échantillons traités étaient dilués au ½ dans du tampon de course avec 10% final de NSB reducer (GE Healthcare).

*Matériels et méthodes pour le Tableau 3*

15000 RU d'anticorps ont été immobilisés pour la piste B2M-01 (classe I), et 18000 RU pour la piste Tu169 (DQ). Les sérums humains utilisés venaient de patients non immunisés anti-HLA inscrits sur liste d'attente de transplantation. Ceux-ci avaient subi un traitement à la chaleur puis une purification des IgG sur protéine G et concentration à deux reprises afin d'obtenir 1,15 ml de purifiat dans du tampon de course, ou bien un traitement à la chaleur suivi d'un traitement au DTT et une dialyse contre du tampon de course. Les anticorps monoclonaux A2OL (pour anti-HLA-A2 One Lambda) et DQ2OL (pour anti-DQ2 One Lambda) ont été dilués au 1/2000 et 1/4000 dans les sérums traités dilués au ½ dans du tampon de course avec 10% final de réducteur de bruit de fond *NSB reducer* (GE Healthcare). Pour l'A2OL, le blanc était réalisé par l'injection de cet anticorps sur la surface où une grande quantité de HLA-A11 était capturée (1103+/-16 RU), et le test lui-même sur une surface où une grande quantité de HLA-A2 était capturée (1014+/-18 RU). Pour le DQ2OL, le blanc était réalisé par l'injection de cet anticorps sur la surface où une grande quantité de HLA-DQ7 était capturée (545+/-26 RU), et le test lui-même sur une surface où une grande quantité de HLA-DQ2 était capturée (523+/-16 RU).

*Matériels et méthodes pour le Tableau 4*

Cinq sérums de patients immunisés anti-HLA DQ2 ou DQ7 ont été traités à la chaleur suivi d'un traitement au DTT et une dialyse contre du tampon de course. Le dosage des IgG a été réalisé dans le sérum avant et après traitement. Le test Single Antigen a été réalisé sur sérum traité à l'EDTA (SAFB MFI). La CFCA a été réalisée sur ces échantillons dilués au ½ dans du tampon de course avec 10% final de NSB reducer. Pour les patients avec des anticorps ciblant le HLA-DQ2, le blanc était réalisé par l'injection de l'échantillon sur la surface où une grande quantité de HLA-DQ7 était capturée, et le test lui-même sur une surface où une grande quantité de HLA-DQ2, et vice et versa pour les patients avec des anticorps ciblant le HLA-DQ7. Les niveaux de capture étaient de 807+/-19 pour HLA-DQ2 et 801+/-23 pour HLA-DQ7, sur une piste où 20000 RU de Tu169 était immobilisés. La concentration corrigée correspond à la concentration présente initialement dans le sérum, obtenue par déduction des dosages d'IgG totales dans les sérums (IgG initiale) et les échantillons traités (IgG finale).

*Matériels et méthodes pour Figure 2*

Un sérum de patient immunisé anti-HLA DQ2 a été traité à la chaleur puis a subi une purification des IgG sur protéine G et concentration à deux reprises afin d'obtenir 1,15 ml de purifiat dans du tampon de course. La CFCA a été réalisée sur cet échantillon dilué au 1/200 dans du tampon de course avec 10% final de NSB reducer. Le blanc était réalisé par l'injection de l'échantillon sur la surface où une grande quantité de HLA-DQ5 (818 +/- 2 RU) était capturée. Le test lui-même a été réalisé sur une surface où une grande quantité de HLA-DQ2 était capturée (790+/-3 RU) par 15000 RU de Tu169 immobilisés.

*Matériels et méthodes pour Figures 3 et 4*

La SCK a été réalisée sur le même échantillon que la Figure 2 à des concentrations déduites de la CFCA, injectées selon cet ordre : 2,16 nM, 10,8 nM et 54 nM pendant 60 s à 25 μl/min, sans régénération entre les injections. Après la troisième injection, une période de dissociation de 400 s a été appliquée. Le blanc a été réalisé par l'injection de l'échantillon sur la surface où une faible quantité de HLA-DQ5 (78+/-0 RU) était capturée. Le test lui-même a été réalisé sur une surface où une faible quantité de HLA-DQ2 était capturée (82,5+/-0,7 RU) par 15000 RU de Tu169 immobilisés.

*Matériels et méthodes pour Tableau 7*

Le traitement appliqué aux sérums était une exposition à la chaleur puis une purification des IgG sur protéine G et concentration à deux reprises afin d'obtenir 1,15 ml de purifiat dans du tampon de course. La CFCA a été réalisée sur ces échantillons dilués du ½ au 1/200 dans du tampon de course avec 10% final de NSB reducer. Le blanc était réalisé par l'injection de l'échantillon sur la surface où une grande quantité de HLA-DQ non reconnu par le sérum du patient était capturée par 10300 RU de Tu169 immobilisés. Le test lui-même a été réalisé sur la même surface où une quantité de HLA-DQ cible était capturée en quantité équivalente à l'antigène utilisé pour le blanc. Les SCK ont été réalisées sur les mêmes échantillons et pistes d'analyse à des concentrations croissantes déduites de la CFCA pendant 60 s à 25 μl/min, sans régénération entre les injections. Après la troisième injection, une période de dissociation de 400 s a été appliquée. Le blanc a été réalisé par l'injection de l'échantillon sur la surface où une faible quantité de HLA-DQ non reconnu par le sérum du patient était capturée. Le test lui-même a été réalisé sur la même surface où une quantité de HLA-DQ cible était capturée en quantité équivalente à l'antigène utilisé pour le blanc.

*Matériels et méthodes pour Figure 6*

Un anticorps anti-IgG2a de souris clone (R11-89, BD Biosciences) a été immobilisé à un niveau de 13 000 RU par chimie de couplage sur une puce CM5. La concentration d'une solution de beta-2 microglobuline (B2m) diluée au 1/10 000 dans du tampon de course (PBS-T) a tout d'abord été mesurée par CFCA capture, en capturant 2400 RU d'un anticorps reconnaissant la B2m (clone B2M-01) et en utilisant comme blanc du tampon de course (« PBS-T »). La mesure de la concentration de la B2m a ensuite été répétée en utilisant la méthode auto-blanc et en la diluant dans 3 milieux complexes différents donnant des niveaux de fixation non-spécifique différents (« sérum 1/1000 », « sérum 1/100 » et « sérum 1/10 »). La condition auto-blanc a consisté en la capture à un niveau équivalent à l'anticorps B2M-01 d'un anticorps d'isotype IgG2a ne fixant pas la B2m (clone OKT3, anticorps anti-CD3, BD Biosciences).

**Revendications**

1.  Méthode pour déterminer dans des échantillons biologiques complexes en résonance plasmonique de surface la concentration active d'un analyte et, facultativement des constantes cinétiques d'interaction de l'analyte avec un ligand comprenant

    - la fourniture d'une puce de résonance plasmonique de surface sur laquelle est immobilisé un agent de capture spécifique d'un ligand de l'analyte ;
    - la capture par l'agent de capture d'un ligand témoin ne liant pas l'analyte à tester ;
    - le passage de l'échantillon sur la puce injecté à un flux déterminé pendant un temps déterminé ;
    - la régénération de la surface;
    - la capture par l'agent de capture d'un ligand liant l'analyte à tester ;
    - le passage de l'échantillon sur la puce injecté à un même flux déterminé pendant un même temps déterminé ;
    - la soustraction du sensorgramme obtenu avec le ligand témoin du sensorgramme obtenu avec le ligand liant l'analyte à tester; et
    - le calcul de la concentration active de l'analyte et, facultativement des constantes cinétiques d'interaction de l'analyte avec le ligand,

et ou le ligand témoin et le ligand liant l'analyte à tester sont de masse similaire et, le ligand témoin et le ligand liant l'analyte à tester sont capturées en quantité équivalente par l'agent de capture.

2. Méthode selon la revendication 1, **caractérisée en ce que** l'échantillon biologique complexe est sélectionné parmi le sérum, le plasma, les urines, les liquides de lavages, une ascite, les éluats de biopsies et les milieux de culture cellulaire.

3. Méthode selon la revendication 2, **caractérisée en ce que** l'échantillon biologique complexe est le sérum ou le plasma.

4. Méthode selon l'une quelconque des revendications 1-3, **caractérisée en ce que** l'échantillon est injectée à au moins deux flux différents et que la concentration active de l'analyte dans l'échantillon biologique complexe est calculée.

5. Méthode selon l'une quelconque des revendications 1-4, **caractérisée en ce que** l'échantillon est injectée à différentes concentrations et que les constantes cinétiques d'interaction de l'analyte avec le ligand dans l'échantillon biologique complexe sont calculées.

6. Méthode selon l'une quelconque des revendications 1-5, **caractérisée en ce que** le couple analyte-ligand est choisi parmi un couple anticorps-antigène, un couple ligand/récepteur, un couple xénobiotique/cible moléculaire.

7. Méthode selon l'une quelconque des revendications 1-6, **caractérisée en ce que** l'analyte est un anticorps anti-HLA, le ligand témoin est un antigène HLA non reconnu par l'anticorps à tester et le ligand liant l'analyte à tester est un antigène HLA cible de l'anticorps à tester.

8. Méthode selon la revendication 7, **caractérisée en ce que** l'échantillon subit un ou des traitements préalables sélectionnées parmi un traitement thermique, un traitement au dithiothréitol (DTT), une étape de purification des IgG sur une résine de protéine G, une étape de concentration de l'échantillon, une étape de dialyse, en particulier avec un seuil de coupure (cut-off) de 100 kDa, et une combinaison de plusieurs de ces traitements.

9. Méthode selon la revendication 8, **caractérisée en ce que** l'échantillon subit préalablement une combinaison d'un traitement thermique et d'une étape de purification des IgG sur une résine de protéine G.

10. Méthode selon l'une quelconque des revendications 8-9, **caractérisée en ce que** la méthode comprend une étape préalable dans laquelle les anticorps anti-HLA dans l'échantillon sont détectés.

**Patentansprüche**

1. Verfahren zur Bestimmung der aktiven Konzentration eines Analyten und fakultativ kinetischer Interaktionskonstanten des Analyten mit einem Liganden in komplexen biologischen Proben mittels Oberflächenplasmonresonanz, umfassend

- Bereitstellung eines Oberflächenplasmonresonanz-Chips, auf dem ein Einfangmittel immobilisiert ist, das für einen Liganden des Analyten spezifisch ist;
- Einfangen eines Kontrollliganden mit dem Einfangmittel, der das zu untersuchende Analyt nicht bindet;
- Durchfluss der Probe auf dem Chip, die in einem festgelegten Flüssigkeitsstrom in einer festgelegten Zeit injiziert wird;
- Regeneration der Oberfläche;
- Einfangen eines Liganden mit dem Einfangmittel, der das zu untersuchende Analyt bindet;
- Durchfluss der Probe auf dem Chip, die in einem gleichen festgelegten Flüssigkeitsstrom in einer gleichen festgelegten Zeit injiziert wird;
- Subtraktion des Sensogramms, das mit dem Kontrollliganden erhalten wird, von dem Sensogramm, das mit dem Liganden erhalten wird, der das zu untersuchende Analyt bindet; und
- Berechnung der aktiven Konzentration des Analyten und fakultativ der kinetischen Interaktionskonstanten des Analyten mit dem Liganden,

und wo der Kontrollligand und der Ligand, der das zu untersuchende Analyt bindet, in ähnlicher Menge vorliegen und der Kontrollligand und der Ligand, der das zu untersuchende Analyt bindet, in äquivalenter Menge von dem Einfangmittel eingefangen werden.

2. Verfahren gemäß Anspruch 1, **dadurch gekennzeichnet, dass** die komplexe biologische Probe aus Serum, Plasma, Urin, Waschflüssigkeiten, Aszites, Eluaten von Biopsien und Zellkulturmedien ausgewählt ist.

3. Verfahren gemäß Anspruch 2, **dadurch gekennzeichnet, dass** die komplexe biologische Probe Serum oder Plasma ist.

4. Verfahren gemäß einem der vorhergehenden Ansprüche 1 bis 3, **dadurch gekennzeichnet, dass** die Probe in wenigstens zwei unterschiedlichen Flüssigkeitsströmen injiziert wird und dass die aktive Konzentration des Analyten in der komplexen biologischen Probe berechnet wird.

5. Verfahren gemäß einem der vorhergehenden Ansprüche 1 bis 4, **dadurch gekennzeichnet, dass** die Probe in unterschiedlichen Konzentrationen injiziert wird und dass die kinetischen Interaktionskonstanten des Analyten mit dem Liganden in der komplexen biologischen Probe berechnet werden.

6. Verfahren gemäß einem der vorhergehenden Ansprüche 1 bis 5, **dadurch gekennzeichnet, dass** das Analyt-Ligand-Paar aus einem Antikörper-Antigen-Paar, einem Ligand/Rezeptor-Paar, einem Xenobiotikum/Zielmolekül-Paar ausgewählt ist.

7. Verfahren gemäß einem der vorhergehenden Ansprüche 1 bis 6, **dadurch gekennzeichnet, dass** das Analyt ein Anti-HLA-Antikörper, der Kontrollligand ein HLA-Antigen ist, das von dem zu untersuchenden Antikörper nicht erkannt wird, und der Ligand, der das zu untersuchende Analyt bindet, ein HLA-Zielantigen des zu untersuchenden Antikörpers ist.

8. Verfahren gemäß Anspruch 7, **dadurch gekennzeichnet, dass** die Probe einer oder mehreren vorausgehenden Behandlungen unterzogen wird, ausgewählt aus einer thermischen Behandlung, einer Dithiothreitol-(DTT)-Behandlung, einem IgG-Reinigungsschritt auf einem Protein G-Harz, einem Konzentrierungsschritt der Probe, einem Dialyseschritt, insbesondere mit einer Ausschlussgrenze (cut-off) von 100 kDa und einer Kombination mehrerer dieser Behandlungen.

9. Verfahren gemäß Anspruch 8, **dadurch gekennzeichnet, dass** die Probe zuvor einer Kombination aus einer thermischen Behandlung und einem IgG-Reinigungsschritt auf einem Protein G-Harz unterzogen wird.

10. Verfahren gemäß einem der Ansprüche 8 bis 9, **dadurch gekennzeichnet, dass** das Verfahren einen vorausgehenden Schritt umfasst, in dem die Anti-HLA-Antikörper in der Probe nachgewiesen werden.

**Claims**

1. A method for determining, by surface plasmon resonance in complex biological samples, the active concentration of an analyte and optionally kinetic constants of interaction of the analyte with a ligand, comprising

   - providing a surface plasmon resonance chip on which a capture agent specific to a ligand of the analyte is immobilized;
   - capture, by the capture agent, of a control ligand that does not bind the analyte to be tested;
   - passage of the sample over the chip, injected at a determined flow rate for a determined duration;
   - regeneration of the surface;
   - capture, by the capture agent, of a ligand that binds the analyte to be tested;
   - passage of the sample over the chip, injected at the same determined flow rate for the same determined duration;
   - subtraction of the sensorgram obtained with the control ligand from the sensorgram obtained with the ligand that binds the analyte to be tested; and
   - calculation of the active concentration of the analyte and optionally kinetic constants of interaction of the analyte with the ligand,

   and wherein the control ligand and the ligand that binds the analyte to be tested are of a similar mass and the control ligand and the ligand that binds the analyte to be tested are captured in equivalent amounts by the capture agent.

2.  The method as claimed in claim 1, **characterized in that** the complex biological sample is selected from serum, plasma, urine, lavage liquids, ascites, biopsy eluates and cell culture media.

3.  The method as claimed in claim 2, **characterized in that** the complex biological sample is serum or plasma.

4.  The method as claimed in claim 1-3, **characterized in that** the sample is injected at at least two different flow rates and that the active concentration of the analyte in the complex biological sample is calculated.

5.  The method as claimed in claim 1-4, **characterized in that** the sample is injected at different concentrations and that the kinetic constants of interaction of the analyte with the ligand in the complex biological sample are calculated.

6.  The method as claimed in claim 1-5, **characterized in that** the analyte-ligand pair is chosen from an antibody-antigen pair, a ligand-receptor pair or a xenobiotic-molecular target pair.

7.  The method as claimed in claim 1-6, **characterized in that** the analyte is an anti-HLA antibody, the control ligand is an HLA antigen not recognized by the antibody to be tested and the ligand that binds the analyte to be tested is a target HLA antigen of the antibody to be tested.

8.  The method as claimed in claim 7, **characterized in that** the sample undergoes one or more prior treatments chosen from a heat treatment, a treatment with dithiothreitol (DTT), a step of purification of the IgGs on a protein G resin, a step of concentration of the sample, a step of dialysis, in particular with a cut-off threshold of 100 kDa, and a combination of several of these treatments.

9.  The method as claimed in claim 8, **characterized in that** the sample previously undergoes a combination of a heat treatment and a step of purification of the IgGs on a protein G resin.

10. The method as claimed in claim 8-9, **characterized in that** the method comprises a prior step in which the anti-HLA antibodies in the sample are detected.

FIGURE 1

\*proG = protéine G

\*\*BE = Tampon d'échange

**FIGURE 2**

Temps (sec)

**FIGURE 3**

Temps (sec)

**FIGURE 4**

**Test**

B2m

Ac anti-B2m
(B2M-01, souris
isotype IgG2a)

Anticorps anti-IgG2a
de souris (R11-89)

**Auto-blanc**

B2m

Ac ne fixant pas la
B2m (OKT3, souris
isotype IgG2a)

Anticorps anti-IgG2a
de souris (R11-89)

**FIGURE 5**

FIGURE 6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2013002717 A

**Littérature non-brevet citée dans la description**

- **VISENTIN et al.** *Talanta,* 2016, vol. 148, 478-485
- **KARLSSON et al.** *J. Immunol. Methods,* 1993, vol. 166, 75-778
- **SIGMUNDSSON et al.** *Biochemistry,* 2002, vol. 41, 8263-8276